# EUROPEAN PATENT SPECIFICATION

(11) **EP 2 360 178 B1**
(45) Date of publication and mention of the grant of the patent: **06.05.2015**
(21) Application number: 10196762.8
(22) Date of filing: 23.12.2010
(51) Int. Cl.: C07K 14/31, C12N 9/20

(54) **Novel organic solvent lipase gene**
Neues, für organische Lösungsmittel tolerantes Lipasegen
Nouveau gène de lipase pour solvant organique

(30) Priority: 30.12.2009 MY 0907040
(43) Date of publication of application: 24.08.2011
(73) Proprietor: Universiti Putra Malaysia, Selangor (MY)
(72) Inventor: Rahman, Raja Noor Zaliha Abd., Selangor (MY); Salleh, Abu Bakar, Selangor (MY); Basri, Mahiran, Selangor (MY); Yunus, Jalimah Binti, Selangor (MY)
(74) Representative: Krauss, Jan

(56) References cited:
- WO-A2-01/34809
- JALIMAH BT YUNUS: "Cloning and Expression of An Organic Solvent Tolerant Lipase from Staphylococcus eidermidis AT2", PhD thesis, Universiti Putra Malaysia , 31 May 2009 (2009-05-31), page 25PP, XP002641999, Retrieved from the Internet: URL:http://psasir.upm.edu.my/5651/1/FBSB_2 009_11_abstract.pdf [retrieved on 2011-06-15] -& DATABASE UniProt [Online] 23 September 2008 (2008-09-23), "SubName: Full=Lipase JY; EC=3.1.1.3;", XP002642000, retrieved from EBI accession no. UNIPROT:B5A5A8 Database accession no. B5A5A8 -& DATABASE EMBL [Online] 1 August 2007 (2007-08-01), "Staphylococcus epidermidis strain AT2 16S ribosomal RNA gene, partial sequence.", XP002642001, retrieved from EBI accession no. EM_PRO:EU021221 Database accession no. EU021221
- LONGSHAW C M ET AL: "IDENTIFICATION OF A SECOND LIPASE GENE, GEHD, IN STAPHYLOCOCCUS EPIDERMIDIS: COMPARISON OF SEQUENCE WITH THOSE OF OTHER STAPHYLOCOCCAL LIPASES", MICROBIOLOGY, vol. 146, 1 January 2000 (2000-01-01), pages 1419-1427, XP002952545, SOCIETY FOR GENERAL MICROBIOLOGY, READING, GB ISSN: 1350-0872
- ZHANG Y-Q ET AL: "Genome-based analysis of virulence genes in a non-biofilm-forming Staphylococcus epidermidis strain (ATCC 12228)", MOLECULAR MICROBIOLOGY, vol. 49, no. 6, 1 September 2003 (2003-09-01), pages 1577-1593, XP002293898, WILEY-BLACKWELL PUBLISHING LTD, GB ISSN: 0950-382X, DOI: 10.1046/J.1365-2958.2003.03671.X -& DATABASE UniProt [Online] 1 March 2003 (2003-03-01), "SubName: Full=Glycerol ester hydrolase;", XP002642002, retrieved from EBI accession no. UNIPROT:Q8CTZ1 Database accession no. Q8CTZ1

### SEQUENCE LISTING

<110> Universiti putra Malaysia
<120> Novel organic solvent Lipase Gene
<130> FB24099
<150> 20097040
   <151> 2009-12-30
<160> 18
<170> PatentIn version 3.5
<210> 1
   <211> 1491
   <212> DNA
   <213> Staphylococcus epidermidis
<400> 1
   acgctggcgg cgtgcctaat acatgcaagt cgagcgaaca gacgaggagc ttgctcctct      60
   gacgttagcg gcggacgggt gagtaacacg tggataacct acctataaga ctgggataac     120
   ttcgggaaac cggagctaat accggataat atattgaacc gcatggttca atagtgaaag     180
   acggttttgc tgtcacttat agatggatcc gcgccgcatt agctagttgg taaggtaacg     240
   gcttaccaag gcaacgatgc gtagccgacc tgagagggtg atcggccaca ctggaactga     300
   gacacggtcc agactcctac gggaggcagc agtagggaat cttccgcaat gggcgaaagc     360
   ctgacggagc aacgccgcgt gartgatgaa ggtcttcgga tcgtaaaact ctgttattag     420
   ggaagaacaa atgtgtaagt aactatgcac gtcttgacgg tacctaatca gaaagccacg     480
   gctaactacg tgccagcagc cgcggtaata cgtaggtggc aagcgttatc cggaattatt     540
   gggcgtaaag cgcgcgtagg cggtttttta agtctgatgt gaaagcccac ggctcaaccg     600
   tggagggtca ttggaaactg gaaaacttga gtgcagaaga ggaaagtgga attccatgtg     660
   tagcggtgga atgcgccgag atatggagga acaccagtgg cgaaggcgac tttctggtct     720
   gtaactgacg ctgatgtgcg aaagcgtggg gatcaaacag gattagatac cctggtagtc     780
   cacgccgtaa acgatgagtg ctaagtgtta gggggtttcc gccccttagt gctgcagcta     840
   acgcattaag cactccgcct ggggagtacg accgcaaggt tgaaactcaa aggaattgac     900
   ggggacccgc acaagcggtg gagcatgtgg tttaattcga agcaacgcga agaaccttac     960
   ccaaatcttg gacatcctct gacccctcta gagatagagt tttccccttc gggggacaga    1020
   gtgacaggtg gtgcatggtt gtcgtcagct cgtgtcgtga gatgttgggt taagtcccgc    1080
   aacgagcgca acccttaagc ttagttgcca tcattaagtt gggcactcta agttgactgc    1140
   cggtgacaaa ccggaggaag gtggggatga cgtcaaatca tcatgcccct tatgatttgg    1200
   gctacacacg tgctacaatg gacaatacaa agggragcga aaccgcgagg tcaagcaaat    1260
   cccataaagt tgttctcagt tcggattgta gtctgcaact cgactatatg aagctggaat    1320
   cgctagtaat cgtagatcag catgctacgg tgaatacgtt cccgggtctt gtacacaccg    1380
   cccgtcacac cacgagagtt tgtaacaccc gaagccggtg gagtaaccat ttggagctag    1440
   ccgtcgaagg tgggacaaat gattggggtg aagtcgtaac aaggtagccg a            1491
<210> 2
   <211> 2226
   <212> DNA
   <213> Staphylococcus epidermidis
<400> 2
   cacgactatg aagcaaatgt ttataattat cacttctaat tataatcaag tgttcatgct 60
   tcttcatcat gtgtaatgaa aaacattttt caatatgtta tcattcattt ttaatattgt 120
   gtatacacat aataattata tgtaagtatt tattattttt aataattcaa aatgtaaagt 180
   ttacaaagtt ttaataaata aactaaagta atttatattt ttatgttaat atatacataa 240
   cagttatatt ttcatctttt ataggggaaa aaacttatag aggtgatttt tttgaaaaat 300
   aataatgaaa caagaagatt tagcattagg aagtacacgg tgggagtcgt gtcaatcatt 360
   actgggatta caatatttgt cagtggtcag catgctcaag ctgctgaaat gacacaatca 420
   tcatcagatt ttaacgaaca gtcacaacaa acagaacaag ttgaacacaa agaagataca 480
   actcatttat catacgaatt gaatcaagag ggtgacacag ctagccaatc aaagactaat 540
   caagagaacc aatctgatga aaatgtacaa aaaaagaata atcaaacaca acaagattca 600
   acacaaacgt caccattaaa tgaccaagaa caaactttaa aggggcaaca atcaaaagac 660
   aatcatgtta ccccaaattc acgtcaggat acatatccaa aaggccaaaa tcaagatgat 720
   aaaggcaaac aacagtttaa agataatcaa cactcacaaa cagaacatca acctaatact 780
   caaaaccaaa ataatgatca agattcatca gataaaaagc aacacccatc tgatcaaact 840
   caagccccat cttcaaaagg aacacaacct aaacaatcac agtctatagg agatagagat 900
   aaaacagtaa aacaaccatc ttctaaagta cacaaaatag gtaatacaaa aactgataaa 960
   acagttaaaa caaatcaaaa aaagcaaaca tcattaactt caccacgcgt tgtgaaatca 1020
   aaacaaacta aacatatcaa tcaacttact gcgcaagctc aatataaaaa tcaatatcca 1080
   gtcgtgtttg tacatggatt tgtaggttta gtcggtgaag attcattcag catgtaccca 1140
   aattattggg gtggtactaa atataacgtg aaacaagaac ttacaaaatt aggttaccga 1200
   gttcacgaag ccaatgtagg agcatttagc agcaattatg accgtgctgt tgaactgtat 1260
   tattatatta aaggtggaag agtagattat ggtgcagcac atgctgcaaa atatggtcac 1320
   aagcgttatg gcagaacata tgaaggcatc atgcctgatt gggaaccagg taaaaagata 1380
   catcttgttg gacatagtat gggtggccaa acgatacgct tgatggaaca ttttttaaga 1440
   aatggaaatc aagaagaaat agattatcaa cgtcaatatg gtggtacggt atctgatttg 1500
   tttaaaggtg gccaagataa catggtgtct acgattacta cattaggaac acctcataat 1560
   ggcacacctg ctgcagataa actagggtcg actaaattta tcaaagatac aattaataga 1620
   attggaaaaa ttggtggaac taaagcgctc gatttagaac taggtttttc tcaatggggc 1680
   ttcaaacaga aacctaatga atcatatgct gaatatgcaa aacgtatagc gaatagtaaa 1740
   gtttgggaga ctgaagatca ggctgtaaat gatttaacaa ctgctggagc agaaaagtta 1800
   aaccaaatga cgacattgaa tcctaatatc gtctatacat catatacagg tgctgcaaca 1860
   catactggac cattaggcaa tgaagtgccg aatattagac aattcccact attcgattta    1920
   acaagtcgtg cgataggtgg agatgataat aaaaatgtca gagtaaatga tggcatagta    1980
   cctgtgtctt cttcactaca tccaagtgat gaagcattta agaaggtagg tatgatgaac    2040
   ctagcaactg acaagggtat ttggcaagtg agacccgtac aatatgattg ggatcatcta    2100
   gatttagtcg gcttagatac tactgattat aagcgaactg gagaagaatt aggtcaattc    2160
   tatatgagta tgataaataa tatgttgaaa gtcgaagagt tagatggtat cacacgtaag    2220
   tagtga                                                               2226
<210> 3
   <211> 643
   <212> PRT
   <213> Staphylococcus epidermidis
<400> 3
   Met Lys Asn Asn Asn Glu Thr Arg Arg Phe Ser Ile Arg Lys Tyr Thr
   1               5                   10                  15
   Val Gly Val Val Ser Ile Ile Thr Gly Ile Thr Ile Phe Val Ser Gly
               20                  25                  30
   Gln His Ala Gln Ala Ala Glu Met Thr Gln Ser Ser Ser Asp Phe Asn
           35                  40                  45
   Glu Gln Ser Gln Gln Thr Glu Gln Val Glu His Lys Glu Asp Thr Thr
       50                  55                  60
   His Leu Ser Tyr Glu Leu Asn Gln Glu Gly Asp Thr Ala Ser Gln Ser
   65                  70                  75                  80
   Lys Thr Asn Gln Glu Asn Gln Ser Asp Glu Asn Val Gln Lys Lys Asn
                   85                  90                  95
   Asn Gln Thr Gln Gln Asp Ser Thr Gln Thr Ser Pro Leu Asn Asp Gln
               100                 105                 110
   Glu Gln Thr Leu Lys Gly Gln Gln Ser Lys Asp Asn His Val Thr Pro
           115                 120                 125
   Asn Ser Arg Gln Asp Thr Tyr Pro Lys Gly Gln Asn Gln Asp Asp Lys
       130                 135                 140
   Gly Lys Gln Gln Phe Lys Asp Asn Gln His Ser Gln Thr Glu His Gln
   145                 150                 155                 160
   Pro Asn Thr Gln Asn Gln Asn Asn Asp Gln Asp Ser Ser Asp Lys Lys
                   165                 170                 175
   Gln His Pro Ser Asp Gln Thr Gln Ala Pro Ser Ser Lys Gly Thr Gln
               180                 185                 190
   Pro Lys Gln Ser Gln Ser Ile Gly Asp Arg Asp Lys Thr Val Lys Gln
           195                 200                 205
   Pro Ser Ser Lys Val His Lys Ile Gly Asn Thr Lys Thr Asp Lys Thr
       210                 215                 220
   Val Lys Thr Asn Gln Lys Lys Gln Thr Ser Leu Thr Ser Pro Arg Val
   225                 230                 235                 240
   Val Lys Ser Lys Gln Thr Lys His Ile Asn Gln Leu Thr Ala Gln Ala
                   245                 250                 255
   Gln Tyr Lys Asn Gln Tyr Pro Val Val Phe Val His Gly Phe Val Gly
               260                 265                 270
   Leu Val Gly Glu Asp Ser Phe Ser Met Tyr Pro Asn Tyr Trp Gly Gly
           275                 280                 285
   Thr Lys Tyr Asn Val Lys Gln Glu Leu Thr Lys Leu Gly Tyr Arg Val
       290                 295                 300
   His Glu Ala Asn Val Gly Ala Phe Ser Ser Asn Tyr Asp Arg Ala Val
   305                 310                 315                 320
   Glu Leu Tyr Tyr Tyr Ile Lys Gly Gly Arg Val Asp Tyr Gly Ala Ala
                   325                 330                 335
   His Ala Ala Lys Tyr Gly His Lys Arg Tyr Gly Arg Thr Tyr Glu Gly
               340                 345                 350
   Ile Met Pro Asp Trp Glu Pro Gly Lys Lys Ile His Leu Val Gly His
           355                 360                 365
   Ser Met Gly Gly Gln Thr Ile Arg Leu Met Glu His Phe Leu Arg Asn
       370                 375                 380
   Gly Asn Gln Glu Glu Ile Asp Tyr Gln Arg Gln Tyr Gly Gly Thr Val
   385                 390                 395                 400
   Ser Asp Leu Phe Lys Gly Gly Gln Asp Asn Met Val Ser Thr Ile Thr
                   405                 410                 415
   Thr Leu Gly Thr Pro His Asn Gly Thr Pro Ala Ala Asp Lys Leu Gly
               420                 425                 430
   Ser Thr Lys Phe Ile Lys Asp Thr Ile Asn Arg Ile Gly Lys Ile Gly
           435                 440                 445
   Gly Thr Lys Ala Leu Asp Leu Glu Leu Gly Phe Ser Gln Trp Gly Phe
       450                 455                 460
   Lys Gln Lys Pro Asn Glu Ser Tyr Ala Glu Tyr Ala Lys Arg Ile Ala
   465                 470                 475                 480
   Asn Ser Lys Val Trp Glu Thr Glu Asp Gln Ala Val Asn Asp Leu Thr
                   485                 490                 495
   Thr Ala Gly Ala Glu Lys Leu Asn Gln Met Thr Thr Leu Asn Pro Asn
               500                 505                 510
   Ile Val Tyr Thr Ser Tyr Thr Gly Ala Ala Thr His Thr Gly Pro Leu
           515                 520                 525
   Gly Asn Glu Val Pro Asn Ile Arg Gln Phe Pro Leu Phe Asp Leu Thr
       530                 535                 540
   Ser Arg Ala Ile Gly Gly Asp Asp Asn Lys Asn Val Arg Val Asn Asp
   545                 550                 555                 560
   Gly Ile Val Pro Val Ser Ser Ser Leu His Pro Ser Asp Glu Ala Phe
                   565                 570                 575
   Lys Lys Val Gly Met Met Asn Leu Ala Thr Asp Lys Gly Ile Trp Gln
               580                 585                 590
   Val Arg Pro Val Gln Tyr Asp Trp Asp His Leu Asp Leu Val Gly Leu
           595                 600                 605
   Asp Thr Thr Asp Tyr Lys Arg Thr Gly Glu Glu Leu Gly Gln Phe Tyr
       610                 615                 620
   Met Ser Met Ile Asn Asn Met Leu Lys Val Glu Glu Leu Asp Gly Ile
   625                 630                 635                 640
   Thr Arg Lys
<210> 4
   <211> 19
   <212> DNA
   <213> artificial
<220>
   <223> 16s forward primer
<400> 4
   gagtttgatc ctggctcag 19
<210> 5
   <211> 20
   <212> DNA
   <213> artificial
<220>
   <223> 16s reverse primer
<400> 5
   cggctacctt gttacgactt 20
<210> 6
   <211> 16
   <212> DNA
   <213> artificial
<220>
   <223> M13 forward primer
<400> 6
   gtaaaacgac gggcag 16
<210> 7
   <211> 17
   <212> DNA
   <213> artificial
<220>
   <223> M13 reverse primer
<400> 7
   caggaaacag ctatgac 17
<210> 8
   <211> 21
   <212> DNA
   <213> artificial
<220>
   <223> F1
<400> 8
   cagtggtcag catgctcaag c 21
<210> 9
   <211> 24
   <212> DNA
   <213> artificial
<220>
   <223> R1
<400> 9
   gctaggttca tcatacctac cttc 24
<210> 10
   <211> 24
   <212> DNA
   <213> artificial
<220>
   <223> F2
<400> 10
   cagaacatat gaaggcatca tgcc 24
<210> 11
   <211> 21
   <212> DNA
   <213> artificial
<220>
   <223> R2
<400> 11
   ccatcaagcg tatcgtttgg c 21
<210> 12
   <211> 21
   <212> DNA
   <213> artificial
<220>
   <223> F3
<400> 12
   cacgtcagga tacatatcca a 21
<210> 13
   <211> 21
   <212> DNA
   <213> artificial
<220>
   <223> R3
<400> 13
   gtctatttga tcccagctga t 21
<210> 14
   <211> 21
   <212> DNA
   <213> artificial
<220>
   <223> R4
<400> 14
   ataacatggt gtctacgatt a 21
<210> 15
   <211> 21
   <212> DNA
   <213> artificial
<220>
   <223> F5
<400> 15
   atatccagtt gtttttgtac a 21
<210> 16
   <211> 24
   <212> DNA
   <213> artificial
<220>
   <223> R5
<400> 16
   cctttacttt cagttgcttc aaca 24
<210> 17
   <211> 21
   <212> DNA
   <213> artificial
<220>
   <223> Fm
<400> 17
   caatcaactt actgcgcaag c 21
<210> 18
   <211> 21
   <212> DNA
   <213> artificial
<220>
   <223> Rm
<400> 18
   cactacttac gtgtgatacc a 21

## Description

### FIELD OF INVENTION

The invention discloses an organic solvent tolerant lipase gene obtained from a microorganism. More particularly the present invention relates to a novel lipase gene produced from *Staphylococcus epidermidis* AT2.

### BACKGROUND OF INVENTION

Lipases are obtained from a variety of sources like plants, animals, bacteria. Among all lipases microbial lipases are the most popular enzymes for industrial use as they are easy to produce and are comparatively stable. Bacterial lipases are glycoproteins, but some extracellular bacterial lipases are lipoproteins. The properties of bacterial lipases - optimum pH, molecular weight, heat stability and substrate specificity- differ from lipase to lipase. Lipases from microorganisms are used in various industries such as food, dairy, pharmaceutical, detergents, textile, biodiesel, and cosmetic industry, and in synthesis of fine chemicals, new polymeric materials, and environment industry (Hassan *et al.,* 2006). *Staphylococcus* lipases are larger enzymes with approximate size of 75 kDa. These lipases are secreted in precursor form and processed by a specific lipase in the extracellular medium, yielding a mature protein.

A second lipase gene named *gehD* in Staphylococcus epidermis was reported by Longshaw et al (Microbiology, 2000). The gehD lipase is a 360 amino acid long mature enzyme with a high degree of conservation in its lipase domain over a different species.

Zhang and colleagues provided a complete sequencing of the genome of the Staphylococcus epidermis strain ATCC 12228 and show a lipase gene as a possible pathogenic factor of the strain (Zhang et al., Molecular Microbiology 2003).

Various Staphylococcus epidermis polypeptides and encoding DNA are disclosed in WO 01/34809. Also disclosed are methods of their use for the treatment of infections. For example antagonists of their function are proposed as therapeutics in the treatment of infectious diseases.

The objective of the present invention is to clone and to express organic solvent tolerant lipases from *Staphylococcus epidermidis* AT2 by using mesophilic hosts. Yet, the invention also discloses a tolerance organic solvent lipase high yield bacterial strain, lipase gene and the lipase application. The bacterial strain sorting naming is *Staphylococcus epidermidis* AT2, preserving registration number is EU 021221, and is gram positive bacterial strain and various organic solvent which can resist certain concentration. The organic solvent-tolerance lipase encode gene produced from the bacterium is separated and cloned by the invention which comprises nucleotide sequence showed as SEQ ID NO:2 and amino acid sequence showed as SEQ ID NO:3. *Staphylococcus epidermidis* AT2 is one of the extremophiles which is can survive and grow in toxic compound (organic solvents). In industrial point of view, if enzymes are naturally stable in the presence of organic solvents and do not require special stabilization, therefore the enzyme will be very useful in an industrial application .

### SUMMARY OF INVENTION

The present invention pertains to the subject matter as claimed in inpendent claim 1 and dependent claims 2 and 3.

The present patent discloses a purified culture of *Staphylococcus epidermidis* strain AT2 that exhibits lipase activity in the presence of an organic solvent. The *Staphylococcus epidermidis* strain AT2 is an organic solvent-tolerant bacterial cell deposited with accession number EU 021221. Moreover, the *Staphylococcus epidermidis* strain AT2 comprises a 16S rDNA nucleotide sequence of SEQ ID NO 1.

Disclosed is a novel organic solvent tolerant lipase gene obtained from *Staphylococcus epidermidis* strain AT2, deposited with accession number EU 814893.The novel organic tolerant solvent lipase gene comprising all or part of SEQ ID No:2 a nucleotide sequence and SEQ ID No 3 an amino acid sequence.

Also disclosed is a method of obtaining identifying from organic solvent-tolerant *Staphylococcus epidermidis* strain AT2, the methods comprising the steps of morphology identification and/ or16S rDNA. The method that includes morphology identification of *Staphylococcus epidermidis* strain AT2 provides the following methods isolating the *Staphylococcus epidermidis* strain AT2 bacteria in a media, obtaining and screening the *Staphylococcus epidermidis* strain AT2 bacteria by performing morphological characterization, extracting genomic DNA, quantifying and purifying the genomic DNA, amplifying a target gene of 16S rDNA using polymerase chain reaction (PCR), obtaining a PCR product from step and purifying the PCR product. Furthermore, the 16S rDNA method of identifying *Staphylococcus epidermidis* strain AT2 comprises the followings amplifying using PCR and sequencing of 16S r DNA obtained from the *Staphylococcus epidermidis* strain AT2, cloning and transforming the 16S r DNA and obtaining positive clones, screening the positive clones and performing plasmid extraction ,obtaining a recombinant plasmid and performing PCR by using the plasmid as a template, sequencing the recombinant plasmid and obtaining a 16S rDNA nucleotide sequence of the organic solvent-tolerant *Staphylococcus epidermidis* strain AT2.

The present disclosure further is extended to a method of obtaining *Staphylococcus epidermidis* strain AT2 organic solvent tolerant lipase gene, wherein the method comprising the provides the followings designing organic solvent tolerant lipase gene from *Staphylococcus epidermidis* strain AT2 primers, isolating the organic solvent tolerant lipase gene, designing organic solvent tolerant lipase gene from *Staphylococcus epidermidis* strain AT2 primers, performing amplification of the solvent tolerant lipase gene through PCR by using the primers, obtaining a PCR product, cloning the PCR product, obtaining a clone comprising the organic solvent tolerant lipase gene, transforming of the gene, performing plasmid isolation, obtaining a recombinant plasmid of organic solvent tolerant lipase and performing PCR by using the plasmid as a template, sequencing the recombinant plasmid and obtaining a nucleotide and amino acid sequence of the organic solvent-tolerant lipase gene, obtaining a nucleotide and amino acid sequence of the *Staphylococcus epidermidis* strain AT2 organic solvent-tolerant lipase gene and analysing target of said lipase gene.

Also, the present disclosure shows AT2 lipase obtained from *Staphylococcus epidermidis* strain AT2, in which the AT2 lipase is capable of having stability in the presence of organic solvents including toluene, *p*-xylene and n-hexane. The AT2 lipase is capable of functioning as a catalyst or as an industrial enzyme.

The present disclosure specifically shows a purified culture of Staphylococcus epidermidis strain AT2 that exhibits lipase activity, wherein the lipase is in the presence of an organic solvent, preferably wherein the Staphylococcus epidermidis strain AT2 is an organic solvent-tolerant bacterial cell deposited with accession number EU 021221. Preferably wherein the AT2 lipase is capable of having stability in the presence of organic solvent(s) including toluene, p-xylene and n-hexane. Also preferred is the above culture wherein the AT2 lipase is capable of functioning as a catalyst or as an industrial enzyme.

A purified culture, wherein the Staphylococcus epidermidis strain AT2 comprising a 16 S rDNA nucleotide sequence of SEQ ID NO 1.

A novel organic tolerant solvent lipase gene obtained from Staphylococcus epidermidis strain AT2 is deposited with accession number EU 814893, preferably wherein the lipase gene comprising all or part of SEQ ID No:2 a nucleotide sequence and SEQ ID No 3 an amino acid sequence.

Disclosed is a method of obtaining identifying from organic solvent-tolerant Staphylococcus epidermidis strain AT2 , wherein the methods comprising the steps of: a) morphology identification and/ or b) 16S r DNA

Preferably wherein the method in step a) further comprises the steps of: a) isolating the Staphylococcus epidermidis strain AT2 bacteria from step (a) in a media, b) obtaining and screening the Staphylococcus epidermidis strain AT2 bacteria by performing morphological characterization, c) extracting genomic DNA, d) quantifying and purifying the genomic DNA from step (d), e) amplifying a target gene of 16S r DNA using polymerase chain reaction (PCR), f) obtaining a PCR product from step and purifying the PCR product.

Furthermore a method of identifying Staphylococcus epidermidis strain AT2 is disclosed, wherein in step b), 16S r DNA, the method further comprises the steps of: a) amplifying using PCR and sequencing of 16S r DNA obtained from the Staphylococcus epidermidis strain AT2, b) cloning and transforming the 16S r DNA from step (a) and obtaining positive clones, c) screening the positive clones from step (b) and performing plasmid extraction, d) obtaining a recombinant plasmid from step (c) and performing PCR by using the plasmid as a template, e) sequencing the recombinant plasmid from step (d) and obtaining a 16S rDNA nucleotide sequence of the organic solvent-tolerant Staphylococcus epidermidis strain AT2.

The disclosure also shows a method of identifying Staphylococcus epidermidis strain AT2 is preferred, wherein the Staphylococcus epidermidis strain AT2 is organic solvent-tolerant. Also preferred is that the 16S r DNA has an amplification size of 1500 base pairs.

Another aspect of the disclosure relates to a method of obtaining Staphylococcus epidermidis strain AT2 organic solvent tolerant lipase gene, wherein the method comprising the steps of: a) designing organic solvent tolerant lipase gene from Staphylococcus epidermidis strain AT2 primers, b) isolating the organic solvent tolerant lipase gene , c) performing amplification of the solvent tolerant lipase gene through PCR by using the primers from step (b), d) obtaining a PCR product from step (c), e) cloning the PCR product from step (d), f) obtaining a clone comprising the organic solvent tolerant lipase gene, g) transforming of the gene from step (f), h) performing plasmid isolation from step (g), i) obtaining a recombinant plasmid of organic solvent tolerant lipase from step (h) and performing PCR by using the plasmid as a template, j) sequencing the recombinant plasmid from step (i) and obtaining a nucleotide and amino acid sequence of the organic solvent-tolerant lipase gene, k) obtaining a nucleotide and amino acid sequence of the Staphylococcus epidermidis strain AT2 organic solvent-tolerant lipase gene and analysing target of said lipase gene.

It is however preferred that the lipase gene has an amplification size of 1666 base pairs.

Comprised by the disclosure is a lipase gene having a sequence at least 75% identical to the sequence according to SEQ ID No.2. However, in certain preferred aspects the lipase gene of the invention has a sequence which is preferably 80%, 85%, 90%, 95%, 96%, 97%, 98% or 99% identical to the sequence according to SEQ ID No. 2.

The lipase gene consists of the sequence according to SEQ ID No. 2, preferably wherein the lipase gene is the gene deposited with the accession number EU 814893.

The lipase gene as disclosed encodes an organic-solvent tolerant lipase enzyme, preferably wherein the lipase enzyme has an amino acid sequence according to SEQ ID No. 3 or a functional part or variant thereof. Functional variants can be confirmed by testing the enzymatic activity of the variant according to the methods as described in the example section of the present invention.

A lipase gene is preferred in context of the present invention, wherein the lipase gene encodes for the enzyme according to SEQ ID No. 3 and has a nucleic acid sequence varying from SEQ ID No. 2 according to the degeneracy of the genetic code. Most preferably the lipase gene according to the invention is obtained from the Staphylococcus epidermidis strain AT2. For example by an isolation method as described herein below.

The lipase gene according to the herein described invention preferably encodes a gene product which exhibits lipase enzyme activity in the presence of organic solvents, preferably in the presence of toluene, p-xylene and/or n-hexane. Thus the novel lipase gene and lipase enzyme of the invention are related to enzymes harbouring a tolerance with respect to organic solvents. In particular the enzyme displays increased stability.

Another aspect of the disclosure relates to a purified culture of Staphylococcus epidermidis strain AT2 that exhibits lipase activity, wherein the lipase is in the presence of an organic solvent. Most preferred is a purified culture of the invention, wherein the Staphylococcus epidermidis strain AT2 is an organic solvent-tolerant bacterial cell deposited with accession number EU 021221.

The purified culture preferably comprises a 16 S rDNA nucleotide sequence according to SEQ ID No. 1.

Also disclosed is a method of obtaining and/or identifying an organic solvent tolerant Staphylococcus epidermidis strain, wherein the methods comprises the steps of:
a) morphology identification and/ or
b) 16S r DNA

### BRIEF DESCRIPTION OF THE DRAWINGS

The present invention will be fully understood from the detailed description given herein below and the accompanying drawings which are given by way of illustration only, and thus are not limitative of the present invention, wherein:
SEQ ID No: 1 depicts the sequence of the 16s rDNA gene identified from a clone of *Ztaphylococcus epidermidis* strain AT2.
SEQ ID No: 2 depicts the nucleic acid sequence of a novel bacterial lipase gene.
SEQ ID No: 3 depicts the amino acid sequence of a novel bacterial lipase gene.
SEQ ID Nos: 4 to 18 depict primer oligonucleotide sequences used in the methods of the present invention.

FIGURE 1 is a representation of Cellular Morphology of *Staphylococcus* sp. AT2 on Nutrient Agar Formed Circular, Entire, Small, Convex and White Colonies on Nutrient Agar after 24 h of Incubation.
FIGURE 2 is a representation of Gram Staining of *Staphylococcus* sp. AT2. Isolate AT2 was shown to be Gram-Positive cocci with grapes shape occur singly, pairs and bunches.
FIGURE 3 is a representation of *Staphylococcus* sp. AT2 on Tributyrin Agar Plate after 24 hours Incubation at 37 °C.A) *Staphylococcus* sp AT2 produced clearing zone on agar plate. B) *Escherichia coli* as negative control.
FIGURE 4 is a representation of *Staphylococcus* sp. AT2 on Rhodamine B Agar Plate after 24 hours Incubation at 37 °C. A) *Staphylococcus* sp AT2 produced orange fluorescent on Rhodamine B agar plate. B) *Escherichia coli* as negative control.
FIGURE 5 is a representation of Genomic DNA Extraction from *Staphylococcus* sp.AT2.
FIGURE 6 is a representation of Amplified 16S rDNA PCR Product.
FIGURE 7 is a representation of 16S rDNA Nucleotide Sequence of *Staphylococcus epidermidis* AT2.
FIGURE 8 is a representation of Rooted Phylogenetic Tree Showing the Relationship of Isolate AT2 to Other *Staphylococcus* spp.
FIGURE 9 is a representation of PCR Product of the Isolated Fragment of Lipase Gene (1666 bp).
FIGURE 10 is a representation of PCR Product of The Isolated Fragment of Lipase Gene.
FIGURE 11 is a representation of Recombinant Clone of AT2/pGem-T easy/ *E. coli* DH5α on Tributyrin-Ampicilin LB Agar Plate after 24 h Incubation at 37°C.
FIGURE 12 is a representation of Amplified PCR Product from Recombinant Plasmid AT2/pGem-T easy/ *E. coli* DH5α.
FIGURE 13 is a representation of PCR Product of the Isolated Fragment of Lipase Gene (1666 bp).
FIGURE 14 is a representation of Nucleotide and Deduced Amino Acid Sequences of The Organic Solvent Tolerant Lipase Gene of *Staphylococcus epidermidis* AT2.
FIGURE 15 is a representation of SignalP Neural Network Output of Organic Solvent Tolerant AT2 Lipase Gene from *Staphylococcus epidermidis* AT2.
FIGURE 16 is a representation of Amino Acid Sequence Alignment of the Mature Domains of *S.*
*epidermidis* AT2 Lipase with those from Other *Staphylococcal* Lipases
FIGURE 17 is a representation of Direct Cloning and Expression of Mature of AT2 Lipase.
FIGURE 18 is a representation of Growth of pTrcHis2/AT2 on Tributyrin- LB Ampicillin Agar Plate.
FIGURE 19 is a representation of Growth of pTrcHis2/AT2 on Rhodamine B-LB Ampicillin Agar Plate.
FIGURE 20 is a representation of Growth of pTrcHis2/AT2 on Triolein-LB Ampicillin Agar Plate.
FIGURE 21 is a representation of SDS-PAGE of AT2 in the Presence of IPTG as Inducer (2) and its Absence.
FIGURE 22 is a representation of Lipase Activity of AT2/TOP10/pTrcHis2 at Different Time Intervals after Induction with 0.60 mM IPTG(▪). AT2/TOP10/ pTrcHis2 Non-induced (◆) and Wild type (▲).
FIGURE 23 is a representation of Different Time Induction with 0.60 mM IPTG.

### BEST MODE TO CARRY OUT THE INVENTION

Before the present invention is further described, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims. When a range of values is provided, it is understood that each intervening value, to the tenth of the unit of the lower limit unless the context clearly dictates otherwise, between the upper and lower limit of that range and any other stated or intervening value in that stated range, is encompassed within the invention.

The upper and lower limits of these smaller ranges may independently be included in the smaller ranges, and are also encompassed within the invention, subject to any specifically excluded limit in the stated range. When the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, the preferred methods and materials are now described. All publications mentioned herein are incorporated herein by reference to disclose and describe the methods and/or materials in connection with which the publications are cited. It must be noted that as used herein and in the appended claims, the singular forms "a," "an," and "the" include plural referents unless the context clearly dictates otherwise.

### EXAMPLES

### Source of Microorganism

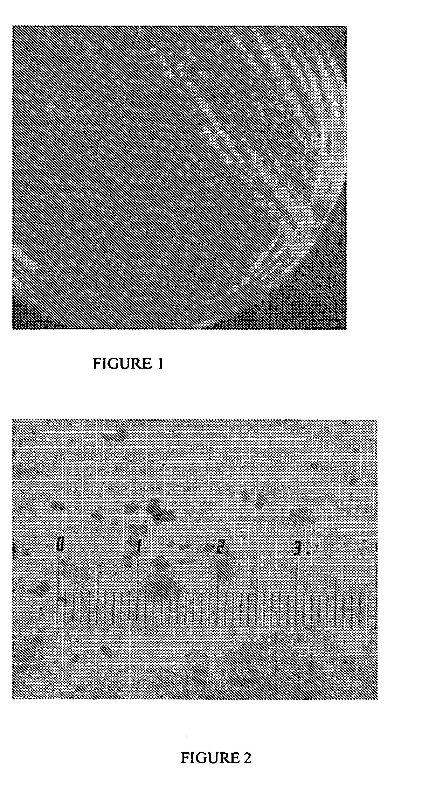

A pure bacteria culture was obtained from stock culture of Enzyme and Microbial Technology laboratories, UPM. This isolate AT2 was tolerant towards Benzane, Toluene, Xylene and Ethylbenzene (BTEX). *Staphylococcus* sp. AT2 was revived from stock culture on nutrient agar plate. Single colonies of *Staphylococcus* sp AT2 was achieved by streak plate method. **Figure 1** showed a pure culture of *Staphylococcus* sp. AT2 on nutrient agar. On the surface of nutrient agar, the isolate AT2 formed round, mucous, small, convex and white colonies. A single colony from 24 h culture was used for Gram staining as young cultures reduced ambiguity of results. The stain culture was observed under light microscope under 100, 400 and 1000 times resolution.

### Screening of Lipolytic Producer

Samples taken were pre-screened qualitatively for their lipase activity. Plates used to detect the presence of lipolytic bacteria acted as differential media. Screening plates used were tributyrin agar prepared according to Oterholm *et al.,* (1968), triolein agar based on Salleh *et al.* (1993) and Rhodamine B agar as said by Kouker and Jaeger (1987).

### Isolation of lipolytic bacteria

Isolation was carried out by selecting colonies of lipolytic bacteria previously screened and each of these colonies were grown and cultured in 10 ml of Tryptic soy Broth for 24 hours at 37 °C. Tryptic soy broth (500 ml); 15g of tryptic soy broth powder is added to 500ml of distilled water prior to sterilization. The cultures were streaked onto various screening plates mentioned above in order to get single colonies. These steps were repeated many times to ensure purity of the isolated lipolytic bacteria colonies. At this stage, the morphology of the isolates was observed on nutrient agar plates. The isolates of the lipolytic bacteria obtained were then made into stock cultures.

### Glycerol Stock Cuture

One single colony of isolated lipase-producing bacteria each was inoculated into aliquots of 10 ml tryptone soy broth. The cultures were incubated at 37 °C overnight, and the bacteria harvested by centrifugation at 12,000 xg and 4 °C for 10 min. Each bacteria pallet was resuspended in a sterilized solution of 15% glycerol in tryptone soy broth (v/v) and vortexed. Aliquots of 0.5 ml were transferred into 1.5 ml Eppendorf tubes and stored at -80 °C.

### Bacterial Identification

### Characterization by Bacterial Morphology

The isolated strain was identified according to the procedure described in the Bergey's Manual of Determinative Bacteriology (Doudoroff and Palleroni, 1974). The bacterium was grown on nutrient agar for 24h. The single colony was picked for Gram staining. The slide was observed under light microscope under 100x, 400x and 1000x resolution. The colonies on the nutrient plate were differentiated by the following morphologies; colony color and size, swarming or spreading, shiny or glossy, sticky and agar color change.

Morphology study showed that the bacteria were Gram-positive cocci with grape shapes, occurred in single, pairs or bunch. **Figure 2** showed gram staining of of 24 h culture of *Staphylococcus* sp. AT2; this bacterium was dark violet or purple in color after decolorization and when safranin was applied, *Staphylococcus* sp. AT2 turned to purple indicating that *Staphylococcus* sp. AT2 was gram positive bacterium coccus

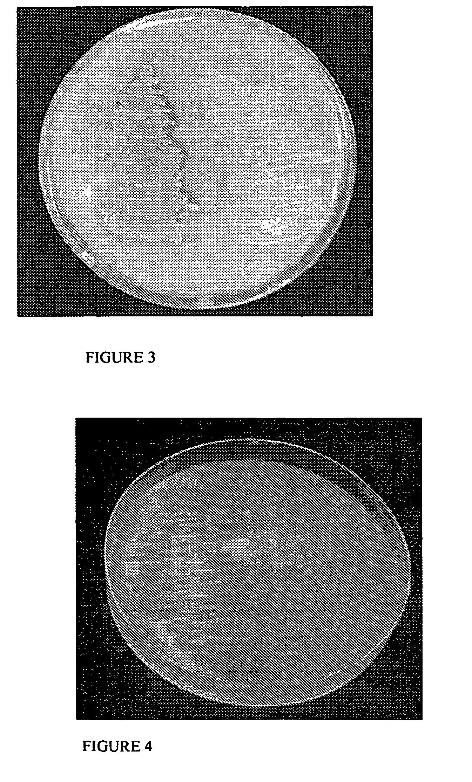

(0.5 X 1.5 µm). The ability of the bacterium to produce lipase was determined qualitatively by growing it on tributyrin agar, triolein agar and Rhodamine B agar overnight at 37 °C. The media contain tributyrin, triolein and Rhodamine B as substrates which can be hydrolyzed by lipase. The formation of the clearing zone around the colonies in tributyrin agar indicated that this bacterium has ability to produce lipase (**Figure 3**). Whereas the exhibition of orange fluorescent is resulted by the formation of Rhodamine B free fatty acid complex between the cationic fluorescent dye Rhodamine B and uranyl-fatty acid (Kouker and Jaeger, 1987). Production of orange fluorescent under ultraviolet radiation is shown in **Figure 4****.**

### Extraction of Genomic DNA

Genomic DNA extraction of *Staphylococcus* sp. strain AT2 was carried out by using the conventional method of Sambrook *et al.,* (1989). Overnight *Staphylococcus* sp. strain AT2 culture was centrifuged at 15000 x g for 10 min at 4 °C. Pellet of bacterial cells was washed twice with 500 µl of GTE buffer [0.2% Glucose, 10 mM Tris-Cl, 1mM EDTA, pH 8.0] and centrifuged at 14000 x g for 10 min. The pellet was resuspended in 300 µl GTE buffer and kept in ice for 5 min. 20 µl of RNase and 50 µl of Lysozymes (10 mg/ml) were added and mixed gently. Then, the mixture was incubated in water bath at 37 °C for 2 hours. After incubation, 50 µl of proteinase K (1 mg/ml) and 50 µl of 25% (w/v) SDS were added and incubated at 50 °C for 30 min. The degraded proteins were removed by adding 500 µl of Phenol:Chloroform:Isoamyl alcohol (PCI) (25:24:1). PCI was added and mixed gently by inverting the mixture for several times. The mixture was centrifuged at 14000 x g for 15 min and two layers were formed. 400 µl of the upper layer was transferred into a new appendorf tube and the DNA was precipitated with 400 µl of sodium acetate (3.0 M, pH 5.5) and 800 µl of isopropanol. The mixture was incubated at room temperature (25 -27 °C) for 10 min, and then centrifuged at 14000 x g for 5 min. The DNA pellet was washed with 500 µl of 80% (v/v) ice cold ethanol followed by centrifuged at 14000 x g for 10 min. The DNA pellet was air dried then dissolved in 50 µl of distilled water and stored at - 20 °C.

### Quantification and Purity of Genomic DNA

Spectrophotometric quantification was carried out to measure the quantity and purity of the extracted genome. 10 µl of extracted genomic material was added and mixed to 990 µl of dH₂O to give final volume of 1000 µl. Absorbance reading was carried out in quartz cuvette at wavelength of 260 nm for genomic concentration. To determine the purity of the DNA, the absorbance reading at 260 nm and 280 nm was taken and the ratio of the absorbance was calculated. The concentration of genomic DNA was calculated according to the formula below:

| |
|---|
| Concentration (µg/ml) = OD 260 x 50 µg/ml x dilution factor |

### Agarose Gel Electrophoresis

Agarose gel electrophoresis was performed with horizontal gel. 1% (w/v) of agarose was dissolved in 1 x Tris-Acetate-EDTA buffer (TAE) and poured into the gel mold to make the horizontal gels. A comb was hanged into the agarose to form wells. After the agarose solidifies, the comb was removed and the gel was placed in container with 1 x TAE buffer covering the gel. The electrophoresis container was connected to the power supply and electrophoresed under a constant voltage at 80 mA for approximately 35 min and then the electrophoresed gel was stained in ethidium bromide (0.5 µg/ml) in dH₂O for 15 min. The gel was placed on UV transilluminator and visualized under UV light.

### Polymerase Chain Reaction (PCR)

The polymerase chain reactions (PCR) were conducted by using genomic DNA as a template to amplify specific gene. The PCR was performed in 100 µl reaction mixture. The mixture containing genomic as template (10 ng/µl, 4.0 µl), 10 X PCR buffer (10.0 µl), 25 mM MgCl2 (6.0 µl), 10 mM deoxynucleotide triphosphatase (dNTP) mix (2.0 µl), forward primer (20 pmol, 2.0 µl) and reverse primer (20 pmol, 2.0 µl), *Taq* DNA polymerase (2.0 µl) and distilled water (72.0 µl). The reactions were carried out for 30 cycles, each cycle with 4 min predenaturation at 94 °C, 1 min denaturation at 94 °C, 2 min annealing at specific temperature based on melting temperature (Tm) of primers, 2 min extension at 72 °C and the final 7 min elongation step at 72 °C for one cycle. The PCR was carried out in GeneAmp PCR system 9600 (Perkin-Elmer). The amplified products were electrophoresed on agarose gel (section 3.3.6).

### Purification of the PCR Product

PCR products were purified by agarose gel electrophoresis (section 3.3.6). Each sample of PCR products (100 µl) were added and mixed with 10 µl of loading dye (6X). The mixtures were loaded into well of agarose gel and electrophoresed at 60 mA for approxiamately 1 hour and 30 min, then stained with ethidium bromide (0.5 µg/ml). Under UV light, the desired fragments were cut with a sterile blade and transferred into sterile appendorf tubes. The samples of PCR products were extracted from agarose gel by using QIAquick Gel Extraction Kit (QIAGEN, Germany) according to the manufacture's instruction. The purified product was sent to First Base Laboratories Sdn Bhd (Shah Alam, Selangor, Malaysia) for sequencing. Before DNA homology search on GenBank database (http://www.ncbi.gov) was performed.

### Competent Cells of Escherichia coli

Competent cells of *Escherichia coli* strain Top 10 was prepared by using rubidium chloride method. The bacteria was revived from stocks by streaking on agar plate, followed by inoculating a single colony in 10 ml LB broth and incubated for 24 hours at 37 °C, 150 rpm. 1 ml of culture was subsequently transferred into 50 ml LB broth and incubated for approximately 4 hours at 37 °C under shaking at 150 rpm. During competent cell preparation, all procedures were handled in ice. When absorbance reading of the culture was around ≈ 0.5 (±0.01) at wavelength 540 nm, the bacterial culture was centrifuged at 3000 rpm, 4 °C for 5 min. The supernatant was then discarded, having the bacterial pellet resuspended in 15 ml of ice cold TFB I [100 mM RbCl₂, 50 mM MnCl₂, 30 mM KAc, 10 mM CaCl₂ and 15% glycerol, pH 5.8 (adjusted with Acetic acid)] and was kept in ice for 1 hour and 30 min, before centrifuging the mixture at 3000 rpm for 5 min, at 4 °C. The bacterial pellet was added and mixed gently with 2 ml of ice cold TFB II [10 mM MOPS (PIPES), 10 mM RbCl₂, 75 mM CaCl₂ and 15% glycerol, pH 6.8 (adjust with KOH)]. Next, the mixture was aliquoted into Eppendorf tube with 100 µl each and the competent cells were stored in -80 °C.

### Bacterial Identification using 16S rDNA Gene Sequence

Isolate AT2 was further identified by using 16S rDNA gene sequence.

### Gene Amplificafion of 16S rDNA

Genomic DNA was used as a template for PCR to amplify a segment of about 1500 bp of the 16S rDNA gene sequence. Universal primers complementary to the conserved region were used. The annealing temperature was at 58 °C based on two universal primers and the primers were:
16S Forward primer: 5'- GAG TTT GAT CCT GGC TCA G-3'
16S Reverse primer: 5'- C GGC TAC CTT GTT ACG ACT T -3'

The 16S rDNA PCR products were electrophoresed on agarose gel and the amplified 16S rDNA PCR products were purified.

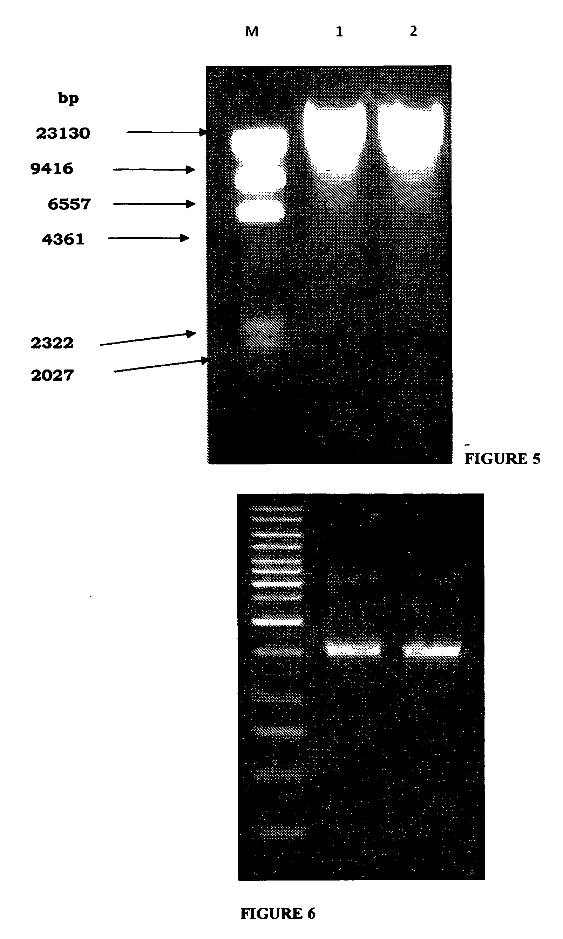

Extracted genomic AT2 was electrophoresed through agarose gel and stained with ethidium bromide (Figure 5). The band was intense in color indicating a high concentration of extracted genomic DNA. The DNA extractions contain pure DNA and eliminating all the cellular components. Pure DNA is important both for proper amplification of PCR and digestion by restriction enzymes.

### Cloning and Transformation of 16S rDNA

Purified 16S rDNA PCR product was cloned into pGem-T easy vector by using PCR Cloning Kit (Promega, USA) according to the manufacturer's instruction. The ligation mixture was incubated for 5 min at room temperature (25-27 °C). The ligation mixture (10 µl) was then transferred into chemically competent *Escherichia coli* strain Top 10 (section 3.3.9) and mixed gently. The mixture was incubated in ice for 30 min and heat shocked for 50 sec at 42 °C without shaking, and was immediately incubated in ice. 250 µl of SOC medium was added and mixed gently. The mixture was subsequently incubated at 37 °C under shaking condition for 1 hour in orbital shaker. After incubation, 100 µl of the mixture was spread on ampicillin LB agar plate with X-gal and incubated at 37 °C for 24 hours.

### Screening for the Positive Clones

Clones were screened on ampicillin LB agar spreaded with X-gal. White colonies were inoculated and grow in ampicillin LB broth for 6 hours and plasmid extraction was done by using QIAprep Miniprep Kit (QIAGEN, Germany) according to the manufacturer's instruction. The recombinant plasmid was electrophoresed on agarose gel and stained with ethidium bromide.

Double digestion of the recombinant plasmid was done by using restriction enzymes. The digestion mixture consists of 5 µl extracted recombinant plasmids, 2 µl of buffer (Y tango) and 1.5 µl of restriction enzymes for final volume of 10 µl. The mixtures were incubated in water bath at 37 °C, for 1 hour for the digestion process to take place and the digestion process was terminated by incubating the digestion mixture at 65 °C for 20 min. The digested plasmids were electrophoresed on agarose gel for analysis.Then, the extracted plasmid was used as a template to run PCR under standard procedure using M13 forward primer and 16S universal reverse primer to double check the insert and orientation of the insert in vector.
M13 Forward primer: 5'- GTA AAA CGA CGG GCA G - 3'
M13 Reverse primer: 5'- CAG GAA ACA GCT ATG AC - 3'

The PCR product was examined under agarose gel electrophoresis and then the positive recombinant plasmids were kept at 4 °C and sent for sequencing.

### Sequencing and Analysis of 16S rDNA Gene Sequence

The recombinant plasmids were sent for automated sequencing using M13 forward and reverse primers. Samples were sequenced using ABI Prism 377 Genetic Analyzer (Perkin-Elmer). The 16S rDNA gene was sequenced separately, generating both forward and reverse (complementary) sequences. The generated 16S rDNA sequence was assembled by aligning the forward and reverse sequences to locate the overlapping between the sequences. To identify the unknown bacterial isolates, 16S rDNA gene sequences of each unknown obtained was subjected to Blast search using NCBI database (www.ncbi.nlm.nih.gov/BLAST). The 16S rDNA sequence was aligned and compared with relevant 16S rDNA sequences currently available in the GenBank database.

16S rDNA gene was amplified with size around 1500 bp as shown in **Figure 6**. Although 500 and 1,500 bp is common lengths to sequence and compare in 16S rDNA, sequencing the 1,500-bp region is necessary to distinguish between particular taxa or strains (Sacchi, 2002). Sequencing of the entire 1,500-bp sequence is also desirable and usually required when describing a new species (Clarridge, 2004).

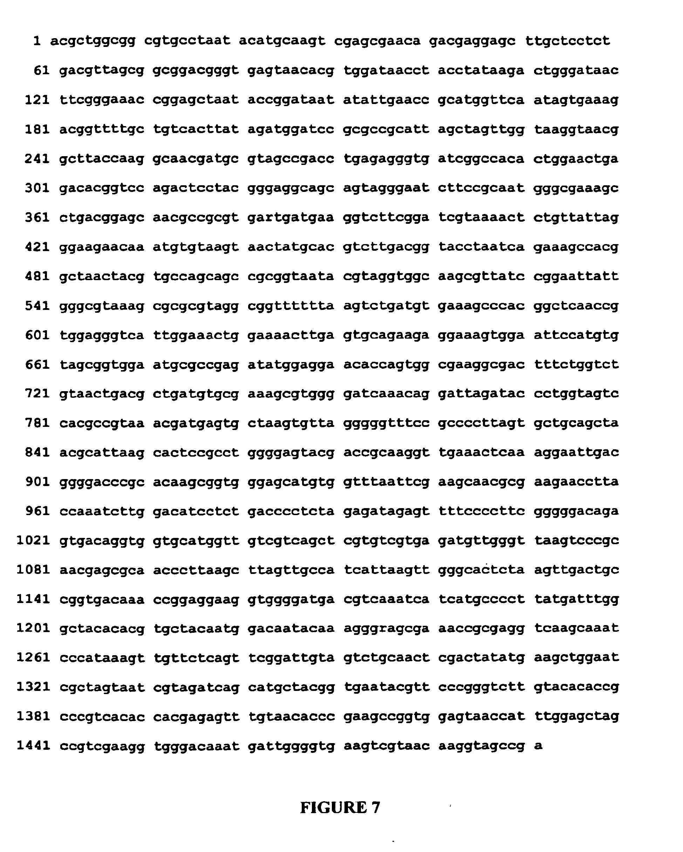

To identify unknown bacterial isolate, the 16S rDNA sequence obtained was subjected to Blast search from the NCBI database (www.ncbi.nim.gov/BLAST). **Figure 7** is the 16S rDNA gene sequence of *Staphylococcus epidermidis* AT2 with length of 1491 bp. The nucleotide sequence of the 16S rDNA gene of *Staphylococcus epidermidis* AT2 in this study has been deposited in the Genbank database under accession no EU021221.

### Phylogenetic Tree Analysis

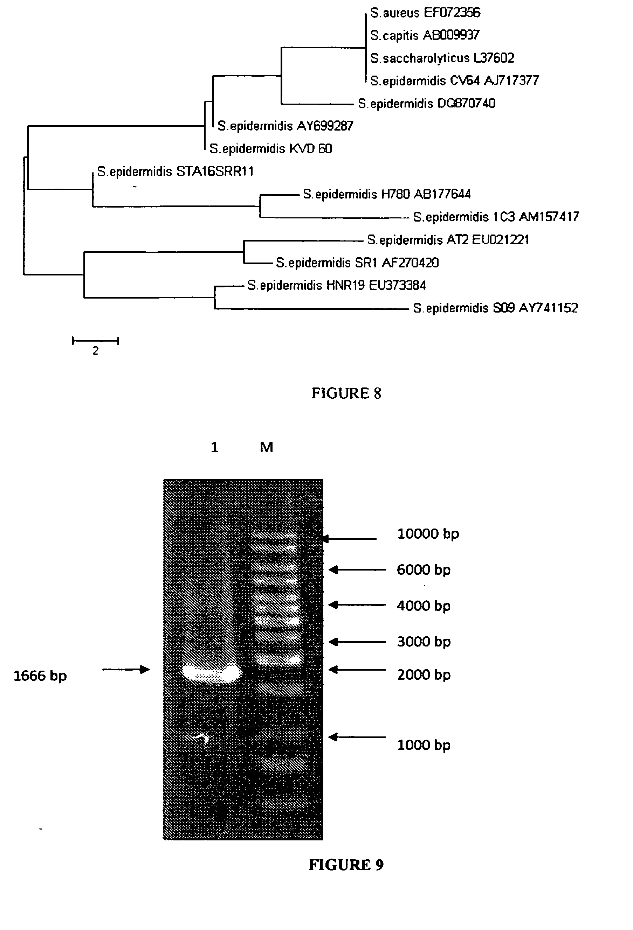

A phylogenetic tree based on comparison of 16S rDNA sequence of strain AT2 and other strains *of Staphylococcus* spp. was constructed (**Figure 8**). All sequences were aligned with CLUSTALW 2. The phylogenetic tree was constructed using Treetop pyhlogenetic Tree Prediction (http://www.genebee.msu.su). 16S rDNA from other *Staphylococcus* spp. were obtained from Genbank database (http:// www.ncbi.nih.gov).

### Cloning and Transformation of organic solvent tolerant lipase via PCR

### Isolation of Organic Solvent Tolerant Gene

Known lipase nucleotide sequences from *Staphylococcus epidermidis* (AF090142) and *Staphylococcus aureas* (AY028918) were obtained from GenBank database and were then aligned with CLUSTALW using software Biology Workbench 2.0 (http://www.workbench.sdsc.edu/). A set of degenerate primer (F₁ and R₁) was designed based on the sequence of conserved region.
F₁: 5' CAG TGG TCA GCA TGC TCA AGC 3'
R₁: 5' GCT AGG TTC ATC ATA CCT ACC TTC 3'

Another two pairs of new primers were designed based on the ORF of *S*. *epidermidis* GehD lipase gene sequence. Primer F₂R₂ was designed based on the downstream sequence of *S. epidermidis* GehD., while primer F₃R₃ was from the upstream of the same sequence.
F₂ 5' CAG AAC ATA TGA AGG CAT CAT GCC 3'
R₂ 5' CCA TCA AGC GTA TCG TTT GGC 3'
F₃ 5' CAC GTC AGG ATA CAT ATC CAA 3'
R₃ 5' GTC TAT TTG ATC CCA GCT GAT 3'

The R₄ was designed to be used with F₂ to amplify the downstream sequence. The oligonucleotide primers F₅ and R₅ were designed to amplify the full length organic solvent tolerant lipase gene.
R₄ 5' ATA ACA TGG TGT CTA CGA TTA 3'
F₅ 5' ATA TCC AGT TGT TTT TGT ACA 3'
R₅ 5' CCT TTA CTT TCA GTT GCT TCA ACA 3'

The PCR reaction was carried out in100 µl of reaction mixture containing 10X PCR buffer, 2 mM MgCl₂, 10 mM dNTP mix, 2 unit of Taq DNA Polymerase, 10 pmol of each forward and reverse primers and genomic DNA as template (50-100ng). The PCR reaction was carried out using the following programmed: 4 min at 94 °C, 30 PCR cycles (94 °C 1min, 45 °C 1min and 72 °C 1min). This followed by 1 cycle of 7 min 72 °C and hold at 4 °C. The reaction was amplified in a thermocycler (GeneAmp PCR system 2400, Perkin Elmer, Foster, CA). The amplified products were examined by agarose gel electrophoresis (3.3.6). The PCR products were further purified using Gel Extraction kit (3.3.8). (Qiagen, USA). The purified product was sent to First BASE Laboratories Sdn Bhd (Shah Alam, Selangor, Malaysia) for sequencing.

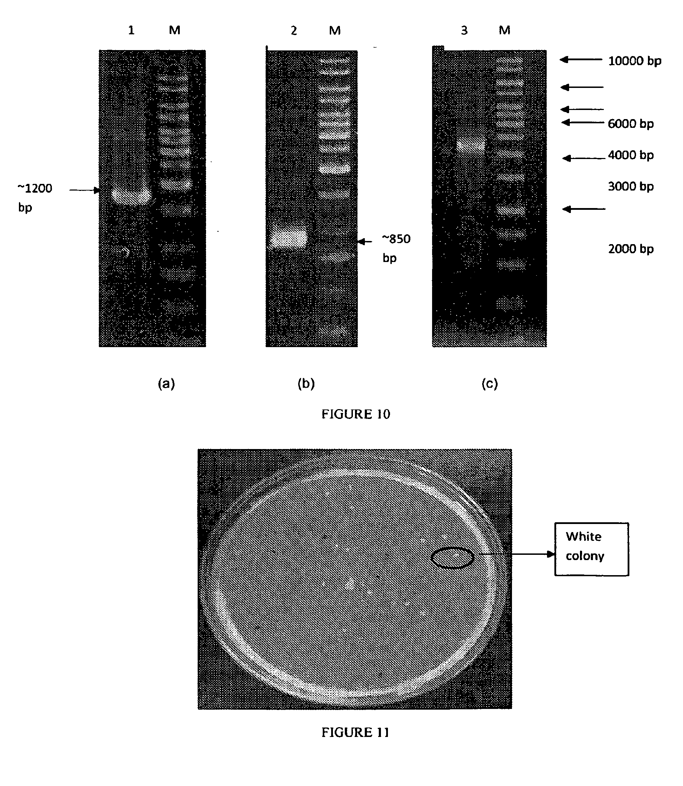

The first set of primer F₁ and R₁ successfully amplified a 1666 bp fragment (**Figure 9**), and the second set F₂ and R₂ amplified at 1.2 kb fragment (**Figure 10a**). The upstream sequence of the open reading frame was amplified by F₃ and R₃, which amplified 1790 bp fragment. The R₄ was designed to be used with F₂ to amplify the downstream sequence of about 850 bp to the stop codon TAA (**Figure 11b**). The oligonucleotide primers F₅ and R₅ were designed to amplify the full length organic solvent tolerant lipase gene which is 2300 bp in size (**Figure 11c**).

### Cloning the PCR product using pGEM-T easy vector

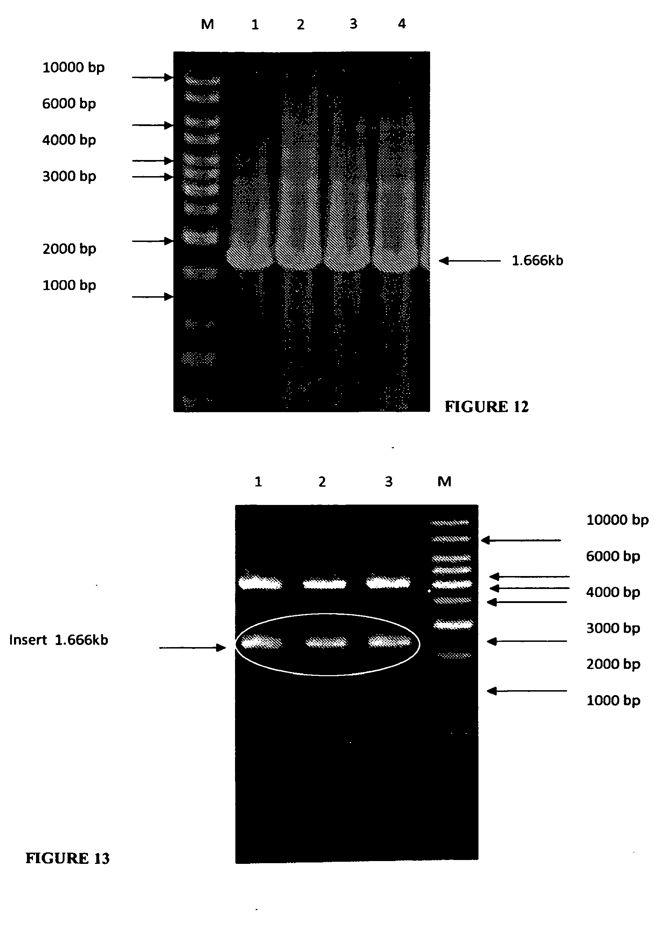

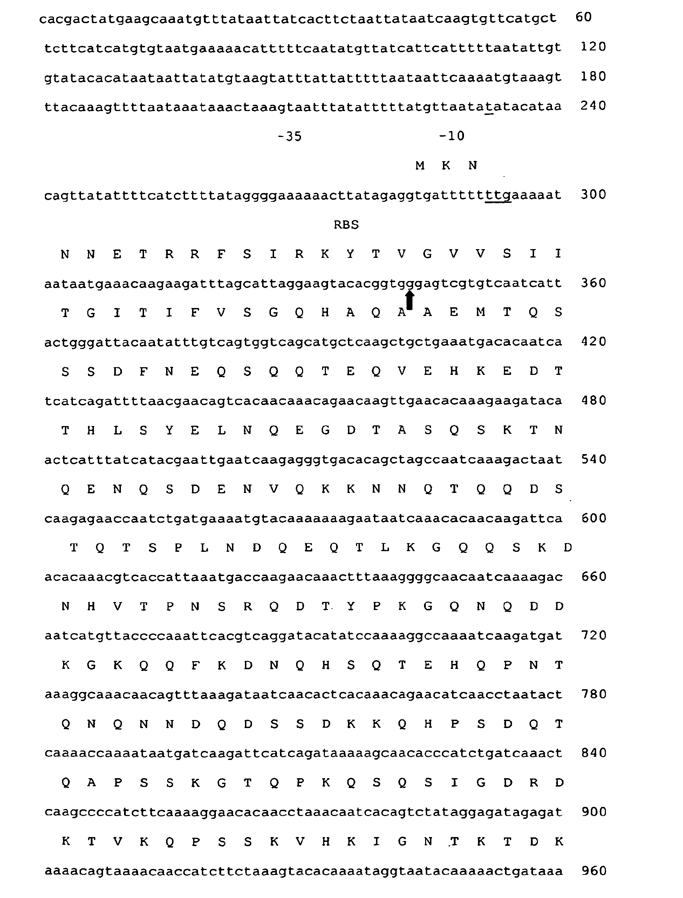

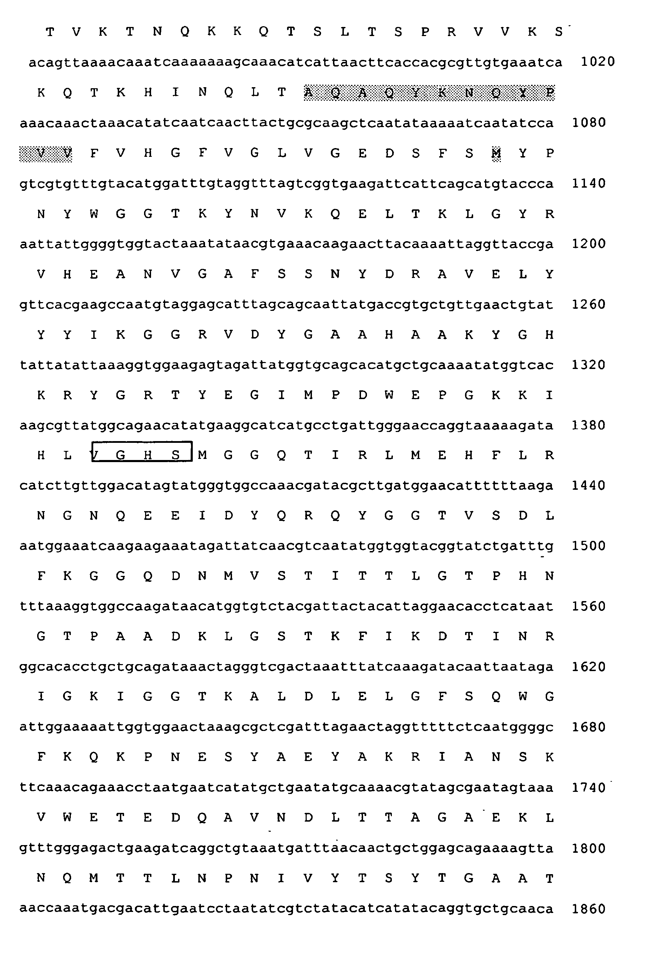

The purified PCR product was cloned into the pGEM-Teasy vector (Promega, USA). The fresh PCR product (3 µl), pGEM-T easy vector (1 µl, 50 ng/µl), 1 µl T4 DNA ligase, 5 µl 2x Rapid ligation buffer and, T4 DNA Ligase were transferred into 0.6 ml PCR tube. The ligation mixture was briefly mixed and incubated at 4 °C overnight in a thermocycler (Gene AMP PCR system 9600 Perkin-Elmer). Nine positive white colonies were extracted to check the size of the plasmids (Figure 11). The plasmids revealed sizes of 4 to 5 kb after gel electrophoresis. The positive plasmids were amplified with primer F₁ and R₁ to confirm the insert. A 1.6 kb of PCR product was successfully amplified (Figure 12).The plasmids from the positive clones were also double digested with restricted enzyme EcoR1 to check the insert (Figure 13). Based on both RE digestion and PCR amplification, it was confirmed that the 1.6 kb insert was successfully cloned into plasmid and the recombination was successful.

### Transformation of E. coli with ligated DNA

The ligated DNA (5 µl) was added to 200 µl of competent cells and kept on ice for 20 min. This mixture was subjected to heat shock for 50-60 s in water bath at 42 °C. The tube was returned immediately to ice for 2 min. After that, 0.9 ml of SOC was added and the mixture shaken at 37 °C for 1.5 h. The transformed cells (100 µl) were spread on pre-warmed LB plates containing tributyrin-LB agar plates containing ampicilin (50-100 µg/ml) and x-Gal, and then incubated overnight at 37 °C.

### Plasmid Isolation

The white transformed colonies grew on LB / ampicillin/ x-Gal plate after overnight incubation at 37 °C were transferred into LB broth containing 50 µg/ml ampicillin. The recombinant plasmids were isolated and purified using the QIAprep Miniprep Kit (Qiagen, Germany). The recombinant plasmid was electrophoresed on agarose gel and stained with ethidium bromide.

### Analysis of the Positive colonies

The presence of the lipase gene (insert) was checked by double digestion. The plasmids were extracted and double digested by using two restriction enzymes. The volume of each reaction mixture was 10 µl, consisting of 1.5 µl *Eco* RI (10 u/µl), 1.5 µl *Bam HI* (10 u/µl), 2.0 µl (10x) Tango buffer, 2.0 µl deionized water and 3 µl extracted plasmids. The reaction mixtures were incubated in a water bath at 37 °C for 2 hours. To detect the plasmid and insert, the recombinant plasmid was electrophoresed on agarose gel and stained with ethidium bromide (section 3.3.6 and 3.3.8). The appearance of two bands under UV light indicated the presence of the insert and vector at their size. Furthermore the presence of the insert was reconfirmed by PCR. The set of primers, (F₁) and (R₁) was used to set up a PCR cocktail reaction of PCR buffer, dNTP, *Taq,* polymerase and the extracted plasmids of expected positive colonies as template under conditions: 4 min at 94 °C, 30 PCR cycles (94 °C 1min, 45 °C 1min and 72 °C 1min). This was followed by 1 cycle of 7 min 72 °C and hold at 4 °C. The PCR product was electrophoresed on agarose gel and stained with ethidium bromide.

### Stock Culture

A colony from the master plate was inoculated in LB broth containing 100 µg/ml ampicillin. The culture was incubated horizontally at 37 °C at 200 rpm, grown until mid-log (A₆₀₀=0.6).Aliquots of 0.85 ml from the culture were then transferred into 1.5 ml Eppendorf tubes and mixed with 0.15 ml sterile glycerol. The aliquots were stored at -80°C.

### Gene analysis

### Nucleotide Sequence Analysis

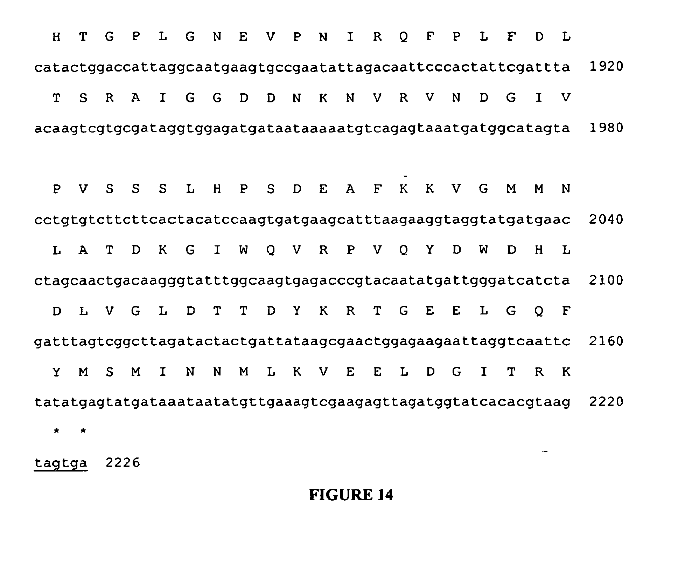

Entire 2.3 kb containing the putative lipase gene was sequenced and was found to contain a single open reading frame (ORF) comprising of 1933 bp extending from 293 to 2226 (**Figure 14**), which codes for 643 amino acids. The deduced molecular mass and pI were calculated to be 72.2 kDa and 8.1, respectively.

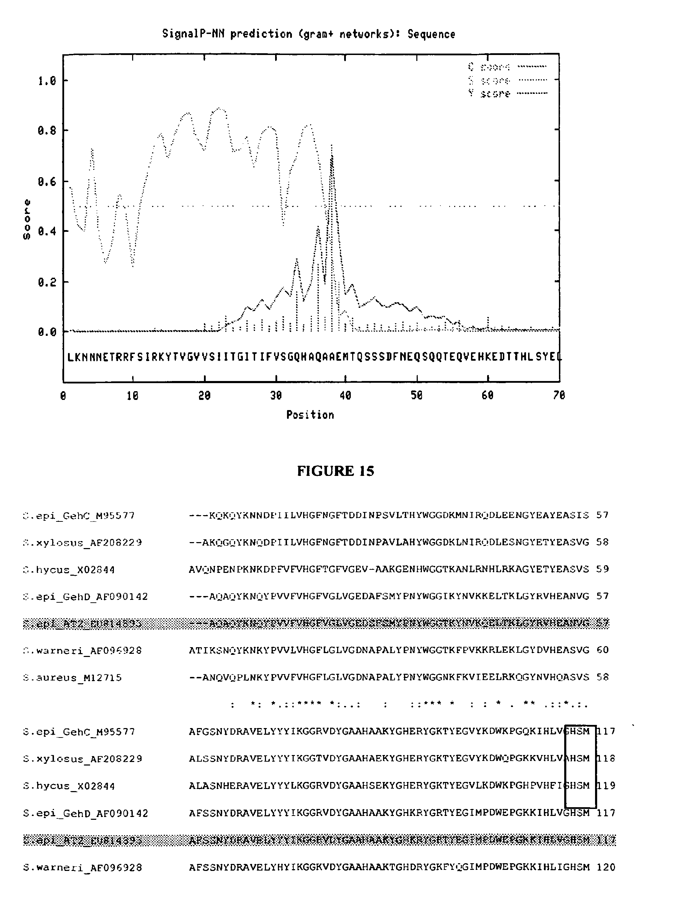

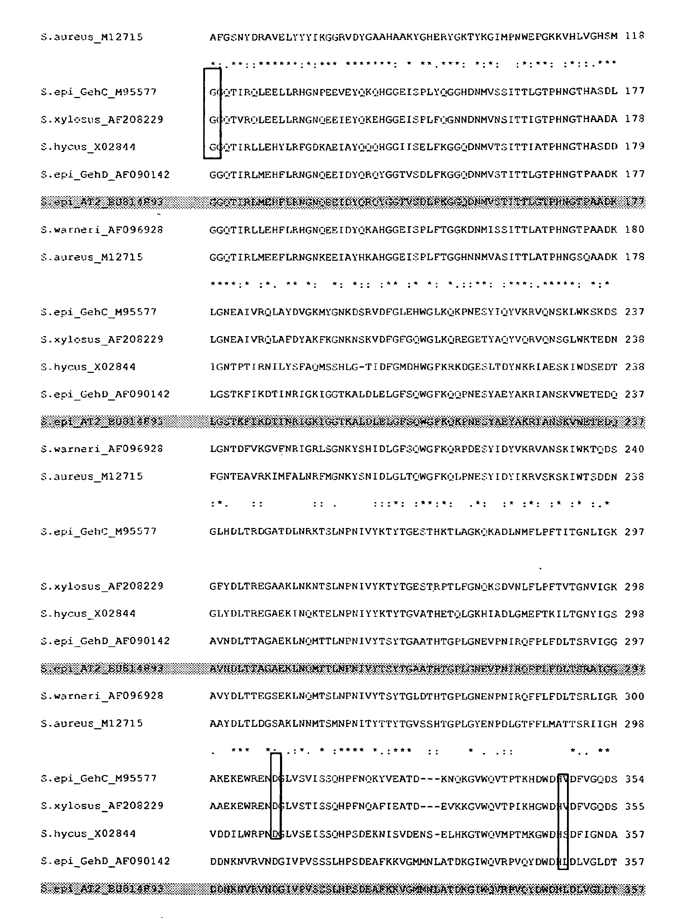

The initiation codon was found to be preceded by a potential ribosome-binding site, AGAGGTG, identical to that found upstream of GehD. In addition, the region of DNA downstream of the predicted translational stop codon contained a palindromic sequence capable of forming a hairpin-loop structure which could act as a transcription terminator (Longshaw *et al.,* 2000). Signal peptides control the entry of proteins to the secretory pathway and they comprise the N-terminal part of the amino acid chain and are cleaved off while the protein is translocated through the membrane (Nielsen *et al.,* 1997). The N-terminal propeptide or signal peptide of *Staphylococcus epidermidis* AT2 lipase consisted of 37 amino acids (Met to Ala). The amino acids sequence which coded the signal peptide are;-'MKNNNETRRFSIRKYTVGVVSIITGITIFVSGQHAQAA-'. The putative signal peptide cleavage site was located at between Ala-37 Ala-38 (**Figure 15**) when predicted by using the SignalP V2.0 World Wide Web server. The complete sequence of the ORF of AT2 lipase gene was submitted to the GenBank and assigned an accession number EU814893.

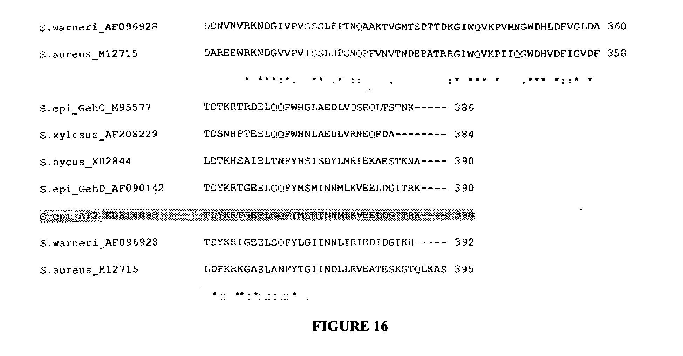

Table 1 shows amino acids compositions of other lipases. Out of the 643 amino acid residues deduced from nucleotide sequence of *S*. *epidermidis* AT2, 75 residues were negatively charge (Asp and Glu) and 77 were positively charge (Arg and Lys) at neutral pH. Charge residues (Arg, Asp, Glu, His and Lys) account for 172 (26.7%) of the amino acid residues in open reading frame of AT2 lipase. AT2 lipase contained 184 hydrophobic residues (Ala, Phe, Ile, leu, Met, Pro, Val, Trp), which corresponded to 28.6% of the total amino acids encountered. The AT2 lipase was classified as a stable protein since its instability index was computed to be 33.75 when calculated using the ProtParam tool in Expasy (http://www.expasy.org/tools). The organic solvent tolerant AT2 lipase consisting of 9982 atoms, is made up of carbon (3127), hydrogen (4899), nitrogen (915), oxygen (1028) and sulfur (13), and is represented by the formula C₃₁₂₇H₄₈₉₉N₉₁₅O₁₀₂₈S₁₃. Aliphatic index of AT2 lipase showed 57.28 based on the deduced sequence of polypeptide chain. Aliphatic index is defined as the relative volume of a protein occupied by aliphatic side chains (alanine, valine, isoloucine and leucine) through statistical analysis (Ikai, 1980). From the amino acids comparison study of eight lipases, it showed that Lys, Gly, Gln and Thr were abundant in these lipases (**Table 1**). On the hand, residues Cys, His, Met, Trp and Phe were less abundant. All lipase have shown a low percentage of Cys molecules if not any. Low numbers of Cys residue are common in lipases. Protein lacking cyctine or with a low content of cysteine are generally more flexible molecules whose tertiary structure relies on weaker bonds. Homology search was performed using BLAST and PSI-BLAST of NCBI. The sequences were aligned with CLUSTALW Multiple Sequence Alignment from Biology Workbench 3.2 (http://workbench.sdsc.edu). The mature amino acid sequence of AT2 lipase was compared with sequences of other well-characterized *Staphylococcal* lipases (**Figure 16**). The mature lipase regions in particular showed a high degree of conservation between all *Staphylococcal* lipase sequences. By comparison of *Staphylococcal* lipase sequences, it became obvious that they have conserved serine, aspartate and histidine residues at positions corresponding to those of the catalytic triad of other lipases.

**Table 1. The amino acid composition and molecular weight of lipases across different families.**

| Amino acid | BAD 90562 | ATCC 12228 | EU 814893 | YP 039776 | YP 189935 | AY 260764 | AF 453713 | AF 034088 |
|---|---|---|---|---|---|---|---|---|
| Ala | 28 | 27 | 28 | 50 | 27 | 33 | 23 | 46 |
| Arg | 21 | 22 | 22 | 25 | 22 | 27 | 7 | 20 |
| Asn | 61 | 46 | 46 | 54 | 44 | 19 | 17 | 8 |
| Asp | 47 | 42 | 42 | 41 | 42 | 23 | 25 | 22 |
| Cys | 0 | 0 | 0 | 0 | 0 | 4 | 1 | 7 |
| Gln | 57 | 58 | 59 | 48 | 60 | 13 | 17 | 18 |
| Glu | 36 | 33 | 33 | 32 | 34 | 20 | 9 | 18 |
| Gly | 48 | 53 | 53 | 52 | 52 | 42 | 25 | 20 |
| His | 26 | 21 | 20 | 27 | 20 | 13 | 7 | 6 |
| Ile | 42 | 26 | 26 | 35 | 27 | 13 | 32 | 7 |
| Leu | 40 | 33 | 33 | 36 | 32 | 40 | 36 | 39 |
| Lys | 69 | 55 | 55 | 57 | 54 | 12 | 28 | 2 |
| Met | 7 | 13 | 13 | 14 | 14 | 8 | 13 | 7 |
| Phe | 23 | 17 | 17 | 17 | 16 | 21 | 17 | 15 |
| Pro | 30 | 23 | 23 | 33 | 22 | 19 | 25 | 19 |
| Ser | 64 | 46 | 46 | 50 | 50 | 27 | 28 | 14 |
| Thr | 43 | 57 | 57 | 52 | 56 | 25 | 22 | 10 |
| Trp | 6 | 6 | 6 | 7 | 6 | 10 | 10 | 3 |
| Tyr | 24 | 26 | 26 | 24 | 26 | 18 | 18 | 10 |
| Val | 38 | 39 | 38 | 37 | 39 | 29 | 37 | 17 |
| | | | | | | | | |
| Total | 711 | 643 | 643 | 691 | 643 | 416 | 397 | 308 |
| MW(Da) | 80085.8 | 72213.6 | 72176.5 | 76691.1 | 72182.5 | 46293.64 | 41802.07 | 33694.95 |

| | | | | | | | | |
|---|---|---|---|---|---|---|---|---|
| Note: The accession numbers of lipases (source origin) were BAD90562(*S*. *warneri*), ATCC12228(S. *epidemidis*), EU814893(S. *epidermidis* AT2), YP039776(S *aureus*), YP189935(*S.epidermidis* RP62A), AY206764(*Geobacillus* sp.T1), AF453713(*Bacillus sphaericus* 205y), AF034088(*Pseudomonas* sp. B11-1). The accession number for AT2 was bold. | | | | | | | | |

### Expression of the Recombinant Organic Solvent-tolerant lipase

### Cloning of the mature lipase gene region

The part of the gene encoding the mature lipase was amplified by PCR from genomic DNA of *S*. *epidermidis* AT2 with a set of degenerated primers was designed based on sequence from *S*. *epidermidis* lipase precursor (GehD) (AF090142).
Fm: 5'- CAA TCA ACT TAC TGC GCA AGC-3'
Rm: 5'-CAC TAC TTA CGT GTG ATA CCA-3'

Amplification process was carried out in reaction mixture containing 50-100 ng DNA template, 30 pmol each forward and reverse primers, 0.2 mM dNTP mix, 2 mM MgCl₂, 2 µl *Taq* DNA polymerase (MBI Fermentas;St. Leon-Rot, Germany), 10x PCR buffer with the following PCR conditions: an initial denaturation step at 94 °C for 4 min, 35 cycles at 94 °C for 1 min, annealing at 45 °C for 1 min, and extension at 72 °C for 1 min, except for the final extension of 7 min, and preservation at 4 °C. The PCR product was electrophoresed using 1% (w/v) agarose gel and purified according to manufacturer's protocols. The purified PCR product of the mature lipase gene region was cloned into expression p*Trc*His2 TOPO vector, and the recombinant plasmids transformed into *E*. *coli* TOP 10. Fresh PCR product (4 µl) and pTrcHis vector (1 µl) were mixed gently and incubated for 5 min at room temperature. The ligated products were transformed with one shot chemically competent *E*. *coli* TOP 10. The mixture was incubated on ice for 30 min and heat-shocked for 30 sec at 42 °C and the tube was then immediately transferred into ice. SOC medium (250 µl) was added before agitation (150 rpm) at 37 °C for 30 min.

The transformed cells were plated onto tributyrin-LB agar plates containing ampicilin (50-100 µg/ml) before underwent incubation at 37 °C overnight. The transformed colonies were positive on triolein and Rhodamine B LB agar plates. Further confirmation was performed by PCR amplification of the insert and double digestion of the plasmids. A transformed colony harboring organic solvent tolerant mature region lipase gene was cultured in LB-ampicilin broth and the plasmids were extracted.

### Plasmid DNA Extraction

Plasmid DNA was extracted by QIAprep Spin Kit by Qiagen. Cells harbouring the plasmid were grown in LB broth containing ampicilin (50 µl/ml) for 16 hours and pelleted by centrifugation. The cell pellet was resuspended in 250 µl of buffer P1. 250 µl of Buffer P2 was added and gently mixed. This followed by addition of 350 µl Buffer N3. The tube was inverted several times before centrifugation at 12,000 X g for 10 min. The supernatant was transferred to the QIAprep column and centrifuged for 60 sec. The flow-through was discarded. QIAprep column was washed by adding 0.5 ml Buffer PB and centrifuged for 30-60 sec and the flow-through was discarded. QIAprep column was washed by adding 0.75 ml Buffer PE and centrifuged for 30-60 sec. The flow-through was discarded and centrifuged for an additional 1 min to remove residual wash buffer. QIAprep column was placed in clean 1.5 ml microcentrifuge tube and DNA was eluted by adding 50 µl Buffer EB (10 mM Tris.Cl, pH 8.5) to the center of QIAprep column, stands for 1 min and followed by centrifugation for 1 min. The sample was loaded onto agarose gel to analyze the size of the extracted plasmid.

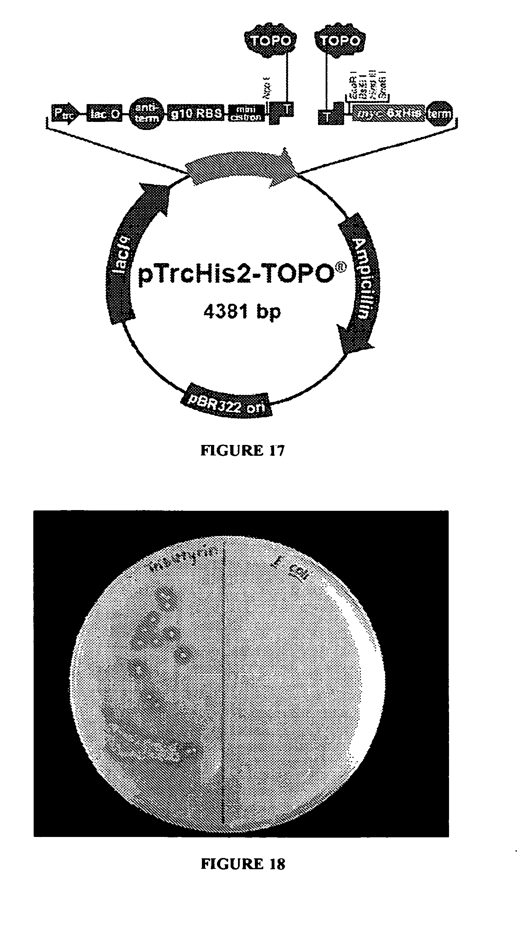

Cloning of AT2 mature lipase gene in pTrcHis2 TOPO vector was conducted in the previous section through PCR cloning. The cloning of PCR products has become standard procedure in molecular biology. In this study, the pTrcHis2 TOPO vector (Invitrogen; Groningen, Netherlands) was used. pTrcHis2 TOPO vector designed for direct cloning of PCR products (**Figure 17**). The mature sequence amplified by Fm and Rm, 21 primers were eluted from the gel and then ligated onto the vector. TOPO cloning technology exploits the fact that *Taq* DNA polymerase and other non-proofreading DNA polymerases add a 3'-end A overhang to the PCR products into the prelinearized pTrcHis2 TOPO vector, which has a U overhang on each 3' end. This eliminates the need for restriction digestion of the vector or insert, primers with built-in restriction site or specially designed adapters, resulting in a much more efficient and robust cloning procedure.

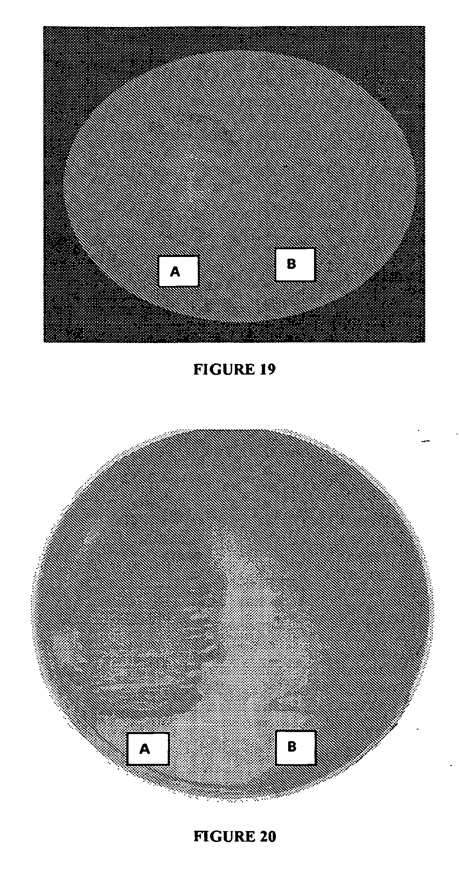

The pTrcHis2 TOPO expression vector (**Figure 17**) allows for ampicillin selection. The vector contains several unique restriction-endonuclease recognition sites around the cloning site allowing easy restriction analysis of the recombinant plasmids. The transformed cells were plated onto tributyrin-LB agar plates (**Figure 18**) containing ampicillin (50-100 µg/ml) before underwent incubation at 37 °C overnight. The transformed colonies were positive on Rhodamine B LB-ampicillin (Figure 19) and triolein LB- ampicillin agar plates (**Figure 20**). Further confirmation was performed by PCR amplification of the insert and double digestion of the plasmids. A transformed colony harboring organic solvent tolerant mature region lipase gene was cultured in LB-ampicillin broth and the plasmids were extracted.

### Optimum IPTG Concentration for Expression

A colony of *E*. *coli* TOP 10 harboring the lipase gene was inoculated into 10 ml LB broth containing 100 µg/ml ampicillin, and grown overnight at 37 °C. Six blue cap media-lab bottles (500 ml) containing 200 ml sterilized LB broth supplemented with 100 µg/ml ampicillin were inoculated with 2 µl of the overnight culture. The cultures were incubated horizontally at 37 °C under shaking at 200 rpm until their growth reached (A_{600 nm}= 0.5). Aliquot of 10 ml from each culture was taken and kept stored at 4 °C and the pellet was left frozen at -20 °C. These aliquots were considered to be zero time. Each culture was subsequently induced with a different IPTG concentration 0.20 mM, 0.40 mM, 0.60 mM, 0.80 mM and 1.0 mM. The cultures were further incubated for 10 h before removing 10 ml aliquots of from each sampled and processed as described above. The pellets from 10 ml aliquots of zero time and 8 h cultures were suspended in 2 ml of 50 mM phosphate buffer. The cells were ultrasonically disintegrated by intermittent use of an UD-200 ultra sonic disruptor at 25W for 2 min in an ice bath. The disrupted cells removed by centrifugation at 12,000 × g and 4 °C for 10 min, and the intra- and extracellular lipase activity were of each culture was assayed according to the method of Kwon and Rhee (1986). The mature lipase gene was cloned and expressed successfully in *E. coli* TOP 10 system using pTrcHis2 as expression vector carrying a *trc* promoter, which could be induced using the lactose analog Isopropyl β-D Thiogalactoside (IPTG). Generally, IPTG is needed to induce RNA polymerase for lipase gene expression. The effect of the IPTG concentration in media has been studied by inducing the cultures with increasing IPTG concentrations. As shown in Table 1, the highest lipase activity (5.3 U/ml) was detected after 8 h induction with 0.60 mM IPTG. The lipase activity without induction with IPTG was 0.32 U/ml and at 0.1 mM of IPTG the activity was 1.6 U/ml, which was enhanced to 500%. The lipase activity was enhanced to more than 50% with 0.4 mM of IPTG. The lipase activity was slightly decreased to 4.2 U/ml at higher concentration of inducer (0.8 mM IPTG) and decreased 100% to 2.1 U/ml at 1.0 mM IPTG. Here, the required concentration of inducer to achieve maximal expression level was determined. The best concentration of IPTG was 0.60 mM, which gave the maximal activity of recombinant AT2 mature lipase.

**Table 2. Lipase activity of AT2 mature/pTrcHis after 8 hours induction at different concentration of IPTG**

| **Concentration of IPTG (mM)** | **Lipase activity (U/ml)*** |
|---|---|
| 0 | 0.32 ± 0.007 |
| 0.1 | 1.6 ± 0.008 |
| 0.2 | 2.5 ± 0.005 |
| 0.4 | 3.2 ± 0.006 |
| 0.6 | 5.3 ± 0.007 |
| 0.8 | 4.2 ± 0.008 |
| 1.0 | 2.1 ± 0.009 |

| | |
|---|---|
| *Data are means ± standard deviation of three determinations. | |

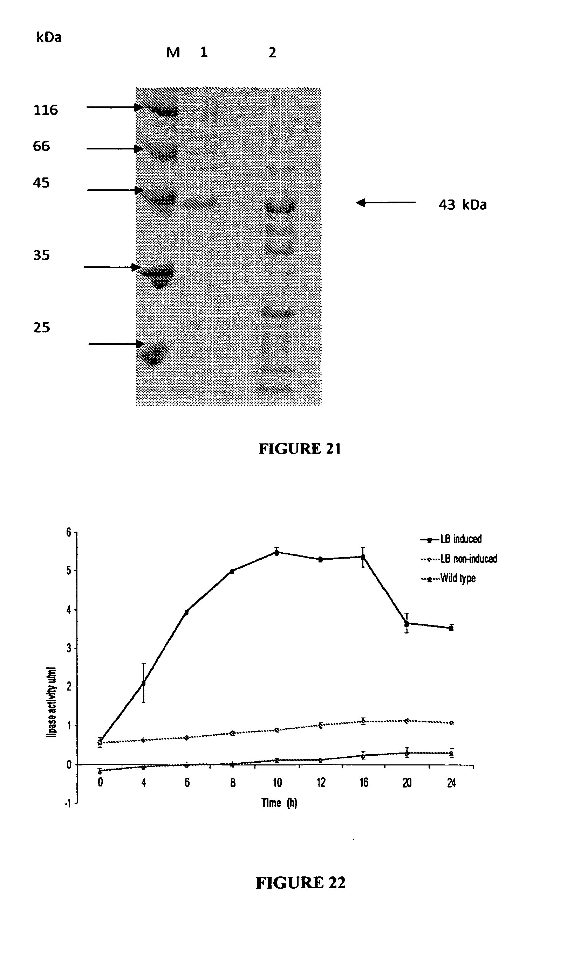

The expression level of AT2 was also determined by SDS-PAGE method. The results from SDS-PAGE showed the bigger band with the presence of IPTG compared to its absence (**Figure 21**). In general, the optimal IPTG concentration when working with *trc* or other derived promoters for the expression of soluble cytoplasmic recombinant proteins has been widely reported to be around 1.00 mM (Durany *et al.,* 2004). This concentration was considered very high for lipase expression. In addition, for production purposes, the amount of IPTG has to be reduced to minimum in order to ensure the economical feasibility.

### Effect of induction time on lipase secretion

*E. coli* TOP 10 harboring recombinant plasmid p*Trc*His2/AT2 were induced with 0.60 mM of IPTG up to 24 h. Induction times ranging from 0-24 h at 4 h intervals were tested for lipase secretion study. The lipase activity was determined intra and extracellular.

### Assay of lipase activity

To analyze intracellular lipase, the cells were harvested by centrifugation at 5,000 x g and 4 °C for 10 min. The pellets were resuspended in 2 ml 50 mM phosphate buffer pH 7.0 and underwent sonication at 25W for 2 min in an ice bath. The disrupted cells were removed by centrifugation at 12,000 x g, 4 °C for 10 min. Liberated free fatty acids were determined colorimetrically according to Kwon and Rhee (1986) using olive oil as substrate. The culture filtrate (1 ml) was shaken with 2.5 ml of olive oil (70% oleate residues) emulsion (1:1, v/v) and 20 ul of 0.02 M CaCl₂ in water bath shaker at an agitation rate of 200 rpm. The emulsion was prepared by mixing together an equal volume of olive oil (Bertoli, Italy) and 50 mM phosphate buffer with a magnetic stirrer for 10 min. The reaction mixture was shaken for 30 min at 37 °C. The enzyme reaction in the emulsion system was stopped by adding 6N HCl (1 ml) and isooctane (5 ml), followed by mixing using a vortex mixer for 30 s. The upper isooctane layer (4 ml) containing the fatty acid was transferred to a test tube for analysis. Copper reagent (1 ml) was added before vortexing for 30 s. The reagent was prepared by adjusting the solution of 5% (w/v) copper (11) acetate-1-hydrate to pH 6.1 with pyridine. The absorbance of the upper layer was read at 715 nm. Lipase activity was measured by measuring the amount of free fatty acid released from the standard curves of free fatty acids. One unit of lipase activity was defined as the amount of enzyme releasing 1 µmole of fatty acid per minute. The free fatty acid released by the hydrolysis action of lipase was determined by measuring the absorbance of isooctane solution at 715 nm. Standard curve of oleic acid (Appendix B) was made in order to measure the amount of free fatty acid to determine the lipase activity. One unit of lipase activity (U) is defined as the amount of enzyme which released 1 µmole of free fatty acid in 1 min at 37 °C. All experiments were done in triplicate and average values were taken. The best incubation time for the highest production of lipase was determined.

Once the optimal IPTG concentration was determined, the optimal time evolution of intracellular recombinant lipase accumulation after induction was investigated. The optimization of the expression of recombinant lipase was carried out by varying the induction times with 0.60 mM IPTG. The recombinant clone was induced with IPTG after the culture growth was reaching OD₆₀₀~0.5. Then, the culture was harvested at different time intervals to determine the optimum induction time for AT2 lipase. The cells were harvested and then lysed by mild sonication and assayed for lipase activity. The lipase activity versus time induction profile is presented in **Figure 22**. At 4 hours after induction, the lipase activity was increased to 2.11 U/ml from 0.36 U/ml at 0 time induction. The lipase activity was increased drastically after 8 hours to about 5.01 U/ml and 5.58 after 10 hours time induction. The recombinant clone reached optimum level of expression at 10 hours after induction with IPTG. At 12 hours induction, the lipase activity was about the same as 8 hours time induction and increased slightly at 16 hours. The expression level decreased gradually at 20 hours and 24 hours with 32% loses in lipase activity. A gradual drop of expression level in a later phase could be explained by the outgrowing of non-induced cells containing plasmid (Hahm *et al.,* 1995).

The expression band was detected on SDS-PAGE stained with comassie blue. The molecular mass of the target protein was determined by comparing with protein marker which was electrophoresed together.

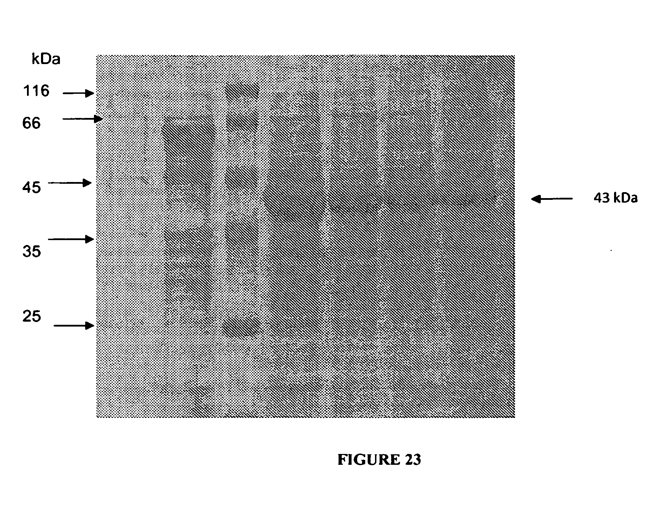

**Figure 23**, shows an expression band of AT2 mature lipase is about 43 kDa on SDS-PAGE. The intensity of expression band was observed at 6, 8 and 10 hours of induction time. Clear correlation was observed between the increases of intensity of expression band proposional with different induction times. AT2 mature lipase gene was successfully cloned and expressed in *E. coli* system with high level of protein expression (~18-mold) compared to wild type. As an overall comparison, the expression level of the recombinant AT2 lipase was higher compared to those reported by Oh *et al.,* (1999) and Drouault *et al.,* (2000). Oh *et al.,* (1999), had reported that the expression of lipase *S*. *haemolyticus* L62 using pBluescript IISK expression vector and *E. coli* XL1 as a host have given low expression level although the N-terminal signal sequence of the expressed preproenzyme was correctly removed. Drouault *et al.,* in (2000) reported that the expression of *S*. *hycus* using *Lactococcus lactis* as expression vector only 10-fold compared to wild type.

### Effect of organic solvents on crude enzyme stability.

The effect of various organic solvents with different Log *P* (Partition Coefficient) value on crude lipase stability was investigated. The cultures harboring recombinant plasmids pTrcHis2/AT2 were induced with 0.6 mM of isopropyl-β-D-thiogalactopyranoside (IPTG) at OD_{600 nm} ~0.5 for 10 h at 37 °C. The culture medium was centrifuged at 10,000 rpm, 4 °C for 10 min. The pallet was then resuspended with 2 ml of potassium phosphate buffer (pH 7.0) and the sonicated with Branson sonifier 250 (output: 2, duty cycle: 30 and 2 min). One ml of organic solvent was added to 3.0 ml of the cell lysate and preincubated at 37 °C, while shaking at 150 rpm for 30 min to ensure the continuous mixing of the enzyme and solvent. The enzyme stability was expressed as the remaining activity assayed according to the method of with Kwon-Rhee (1986) relative to the control value. For control distilled water was added instead of solvent.

AT2 lipase was identified as an organic solvent tolerant enzyme. In order to confirm that the organic solvent tolerant lipase gene was successfully cloned, the stability of TOP 10/AT2/pTrcHis2 lipase in the presence and absence of organic solvents was determined. The enzyme was treated for 30 min in 25% (v/v) various organic solvents with log *P* values from -1.3 to 3.6 and assayed for lipase activity.

As shown in **Table 3**, the lipase had enhanced in the presence of DMSO, p-xylene, n-hexane, toluene and octanol being the most enhancing (- 190% increase activity). The enzyme activity was activated in the present of organic solvents tested with log *P* values of 2.0 to 3.6. Most the activity of the recombinant organic solvent tolerant AT2 lipase was inactivated in the presence of hydrophilic solvents (-1.22 < log *P*_{o/w} < 0). However, the enzyme activity was activated in toluene, octanol, p-xylene and n-hexane with log *P* between 2.0 to 2.5.

**Table 3. AT2 lipase activity in various organic solvents**

| **Solvent** | **Log *P**** | **Relative activity (%)** |
|---|---|---|
| Control | - | 100 |
| Dimethylsulfoxide | -1.3 | 112 |
| Methanol | -0.76 | 1.2 |
| Acetonitrile | -0.33 | 0 |
| Ethanol | -0.24 | 0 |
| Acetone | -0.23 | 0 |
| Diethylether | 0.68 | 0 |
| Ethylacetate | 0.68 | 15 |
| Chloroform | 2.0 | 14 |
| Benzene | 2.0 | 56 |
| Toluene | 2.5 | 230 |
| Octanol | 2.9 | 295 |
| Ethylbenzene | 3.1 | 58 |
| p-xylene | 3.1 | 190 |
| n-hexane | 3.6 | 154 |

| | | |
|---|---|---|
| * Adapted from Laane et al., 1987 Note: The enzyme and organic solvent was mixed in 3:1 ratio and the mixture was incubated at 37°C with shaking at 150 rpm for 30 min and assayed for the remaining lipase activity. | | |

From the previous study (Tajuddin, 2006), the crude enzyme of AT2 lipase was very stable to various organic solvents tested. The crude enzyme exhibited higher tolerance to methanol, 1-dedocanol and 1-heptanol after 30 min exposure to these solvents. The stability and activation effects of the organic solvent tolerant AT2 lipase in aqueous-organic mixtures suggested the ability of this enzyme to resist denaturation by organic solvents. Therefore, the AT2 lipase is naturally stable in organic solvent and holds potential for used in organic synthesis and related application.

It is well known that the effect of organic solvents on enzyme activity differed from lipase to lipase (Sugihara *et al.,* 1992). Therefore, different organic solvents showed different tolerance profiles of AT2 lipase.

### PAGE Electrophoresis

Polyacryamide gel electrophoresis was carried out by using Mini-PROTEAN 3 cell electrophoresis system from Bio-Rad with gel size of 0.75 mm x 8.0 cm x 7.3 cm. The electrophoresis steps including sample buffer preparation, polyacrylamide gel casting, electrophoresis of sample and gel staining in staining solution [62.5 ml of 1% (w/v) Coomassie brilliant blue, 250 ml of 100% (v/v) methanol, 50 ml of 100% (v/v) acetic acid and 137.5 ml of dH₂O] for 10 min and destained in destaining solution [ratio 1:1:8 of 100% (v/v) methanol: 100% (v/v) acetic acid: dH₂O] for 1-2 hour to visualize and analyze the protein band. In polyacrylamide gel casting, Acrylamide and cross linker N,N'-methylene-bis-acrylamide (Bis-acrylamide) were the components of polyacrylamide gel matrix. Stock solution of acrylamide/(bis-acrylamide) was prepared in concentration of 30% T, 2.67% C. Thus, 29.2 g of acrylamide and 0.8 g bis-acrylamide were dissolved in 100 ml dH₂O, filtred and stored at 4 °C in the dark. Buffer Tris-Cl (1.5 M, pH 8.8), buffer Tris-Cl (0.5 M, pH 6.8) and 10% (w/v) SDS were used in gel casting prepared in 100 ml. Freshly prepared ammonium persulphate (APS) (w/v) 10% in 1 ml dH₂O and N,N,N'N'-tetramethyl ethylenediamine (TEMED) added immediately prior to pouring the gel.

### Native PAGE Electrophoresis

Protein sample was diluted with native PAGE sample buffer [1.25 ml of Tris-Cl (0.5 M, pH 6.8), 3.0 ml of 100% (v/v) glycerol, 0.2 ml of 0.5% (w/v) bromophenol blue and 5.55 ml of dH₂O] with ratio of 1:2, thus 50 µl of sample was added to 100 µl of sample buffer. For native polyacrylamide gel casting, the ingredients were listed in Appendix C. 15 µl of protein in sample buffer was applied to each well of the gel and electrophoresed in native running buffer [g/l: Tris-Cl; 3.0 and Glycine; 14.4]. The electrophoresis was run under a constant voltage at 90 volt (13.5 mA) for approximately 3 hours. The electrophoresed gel was stained with Coomassie brilliant blue and destained.

### SDS PAGE Electrophoresis

Preparation of sample in reducing SDS sample buffer [1.25 ml of Tris-Cl (0.5 M, pH 6.8), 2.5 ml of glycerol 100% (v/v), 2.0 ml of SDS 10% (w/v), 0.2 ml of bromophenol blue 0.5% (w/v) and 3.55 ml dH₂O] was carried out by adding 50 µl β-Mercaptoethanol to 950 µl sample buffer prior to used. 50 µl of sample was diluted with 100 µl of sample buffer and followed by heating at 95 °C for 10 min. Appendix D is the list of the ingredients for SDS polyacrylamide gel casting. Each well of gel was loaded with 15 µl of protein in sample buffer and run in running buffer [g/L of Tris; 3.0, glycine; 14.4, and SDS; 1.0] under a constant voltage at 210 volt (30 mA) for approximately 1 hour and 15 min. The gel was stained with Coomassie brilliant blue and destained.

## Claims

1. The use of a lipase enzyme according to SEQ ID No. 3, in the presence of an organic solvent, selected from toluene, p-xylene and/or n-hexane.

2. The use according to claim 1, wherein the lipase is encoded by a gene consisting of the sequence according to SEQ ID No. 2.

3. The use according to any one of the preceding claims, wherein the lipase gene is obtained from the *Staphylococcus epidermidis* strain AT2.

## Patentansprüche

1. Die Verwendung eines Lipaseenzyms gemäß SEQ ID Nr. 3, in Gegenwart eines organischen Lösungsmittels ausgesucht aus Toluen, P-Xylen und/oder n-Hexan.

2. Die Verwendung gemäß Anspruch 1, worin die Lipase durch ein Gen bestehend aus einer Sequenz gemäß SEQ ID Nr. 2 kodiert ist.

3. Die Verwendung gemäß eines jeden der vorstehenden Ansprüche, worin das Lipasegen von dem *Staphylococcus epidermidis* Stamm AT2 erhalten wird.

## Revendications

1. Utilisation d'une enzyme lipase selon la séquence SEQ ID N° 3, en présence d'un solvant organique, choisi parmi le toluène, le p-xylène et/ou le n-hexane.

2. Utilisation selon la revendication 1, dans laquelle la lipase est codée par un gène constitué de la séquence selon la séquence SEQ ID N° 2.

3. Utilisation selon l'une quelconque des revendications précédentes, dans laquelle le gène de lipase est obtenu à partir de la souche *Staphylococcus epidermidis* AT2.
